# EUROPEAN PATENT SPECIFICATION

(11) **EP 1 305 421 B1**
(45) Date of publication and mention of the grant of the patent: **01.03.2006**
(21) Application number: 01959465.4
(22) Date of filing: 03.08.2001
(51) Int. Cl.: C12N 15/31, C07K 14/24, C07K 14/26, C12P 7/60, C12R 1/01, C12R 1/185, C12R 1/22

(54) **ENHANCED 2-KETO-L-GULONIC ACID PRODUCTION**
ERHÖHTE HERSTELLUNG VON 2-KETO-L-GULONSÄURE
PRODUCTION AMELIOREE D'ACIDE 2-CETO-L-GULONIQUE

(30) Priority: 04.08.2000 US 633294; 29.09.2000 US 677032
(43) Date of publication of application: 02.05.2003
(73) Proprietor: GENENCOR INTERNATIONAL, INC., Palo Alto, California 94304 (US); Microgenomics, Inc., Carlsbad, CA 92008 (US)
(72) Inventor: KUMAR, Manoj, Fremont, CA 94555 (US); VALLE, Fernando, Burlingame, CA 94010 (US); DARTOIS, Veronique, A., Delmar, CA 92014 (US); HOCH, James, A., La Jolla, CA 92037 (US)
(74) Representative: Kiddle, Simon John
(86) International application number: PCT/US2001/024327
(87) International publication number: WO 2002/012528

(56) References cited:
- WO-A-00/22170
- WO-A-97/25432
- ANDERSON S ET AL: "Production of 2-keto-L-gulonate, an intermediate in L-ascorbate synthesis, by a genetically modified Erwinia herbicola" SCIENCE, vol. 230, no. 4722, 11 October 1985 (1985-10-11), pages 144-149, XP002031456 ISSN: 0036-8075
- DATABASE EMBL [Online] EMBL; 2 July 1998 (1998-07-02) HAMOOD ET AL.: "Pseudomonas aeruginosa PtxR (ptxR) and PtxS (ptxS) genes, complete cds and ketogluconate utilization operon gene, complete sequence" Database accession no. AF012100 XP002204795 -& SWANSON B L ET AL.: "Characterization of the 2-ketogluconate utilization operon in Pseudomonas aeruginosa PAO1" MOLECULAR MICROBIOLOGY, vol. 37, no. 3, August 2000 (2000-08), pages 561-573, XP002204788
- BOUDRANT J: "Microbial processes for ascorbic acid biosynthesis: a review" ENZYME AND MICROBIAL TECHNOLOGY, vol. 12, no. 5, 1 May 1990 (1990-05-01), pages 322-329, XP000646789 ISSN: 0141-0229
- SAITO Y ET AL.: "Direct fermentation of 2-Keto-L-gulonic acid in recombinant Gluconobacter oxydans." BIOTECHNOLOGY AND BIOENGINEERING, vol. 58, no. 2-3, 20 April 1998 (1998-04-20), pages 309-315, XP002204789 ISSN: 0006-3592
- IZU H ET AL.: "Characterization of the gntT gene encoding a high-affinity gluconate permease in Escherichia coli" GENE, vol. 199, no. 1-2, 15 October 1997 (1997-10-15), pages 203-210, XP004126382 ISSN: 0378-1119
- BAUSCH C ET AL.: "Sequence analysis of the GntII (subsidiary) system for gluconate metabolism reveals a novel pathway for L-idonic acid catabolism in Escherichia coli." JOURNAL OF BACTERIOLOGY, vol. 180, no. 14, July 1998 (1998-07), pages 3704-3710, XP002204790 ISSN: 0021-9193
- KLEMM P ET AL.: "The gntP gene of Escherichia coli involved in gluconate uptake." JOURNAL OF BACTERIOLOGY, vol. 178, no. 1, 1996, pages 61-67, XP002204791 ISSN: 0021-9193
- PEEKHAUS N ET AL.: "Characterization of a novel transporter family that includes multiple Escherichia coli gluconate transporters and their homologues." FEMS MICROBIOLOGY LETTERS, vol. 147, no. 2, 1997, pages 233-238, XP002204792 ISSN: 0378-1097
- CHOTANI G ET AL.: "The commercial production of chemicals using pathway engineering." BIOCHIMICA ET BIOPHYSICA ACTA, vol. 1543, no. 2, December 2000 (2000-12), pages 434-455, XP004279117 ISSN: 0006-3002
- YEW W S ET AL.: "Utilization of L-ascorbate by Escherichia coli K-12: Assignments of functions to products of the yjf-sga and yia-sgb operons." JOURNAL OF BACTERIOLOGY, vol. 184, no. 1, January 2002 (2002-01), pages 302-306, XP002204794 ISSN: 0021-9193
- SWANSON B.L. ET AL: 'Characterization of the 2-ketogluconate utilization operon in Pseudomonas aeruginosa PAO1' MOLECULAR MICROBIOLOGY vol. 37, no. 3, August 2000, pages 561 - 573, XP002204788

## Description

### FIELD OF THE INVENTION

The present invention generally relates to enhancing the industrial production of 2-KLG and specifically to the overexpressing of genome encoding the protein transporting 2,5-diketoglutarate from the periplasm to the cystolic region of the cell. The present invention provides expression vectors, methods and systems for the enhanced production of a 2-KLG in microorganisms.

### BACKGROUND OF THE INVENTION

It is generally well known that 2-keto-L-gulonic acid (2-KLG) is readily converted to L-ascorbic acid (vitamin c) by a one-step chemical procedure in the Reichsteln method (Relchstein, T., et al, Helv. Chim Acta, 1934, 17 311-328; Reichstein, T., et al, 1933, Helv Chim Acta, 16, 561, 1019). There are recombinant microorganisms which express heterologous enzymes to convert a starting substrate to 2-KLG. Recombinant DNA techniques have been used to bioconvert D-glucose to 2-KLG in Erwinia herbicola in a single fermentive step (Anderson, S., et al Science 230, 144-149 (1985)). However, this study is directed to increasing the expression of the 2,5 DKG reductase or other synthetic production without recognizing the importance of substrate transport in the industrial production of the end-product. Indeed, studies by the inventors revealed a minimal increase In the production of 2-KLG as a result or the overexpression of the reductase. Thus there is a need for a means to increase the industrial production of 2-KLG through biosynthetic pathways utilizing recombinant microorganisms by means other than increasing the expression level of the converting proteins within the cell.

The lipid bilayer of biological membranes is generally impermeable to ions and polar molecules. These biological membranes compartmentalize a cell, separating different sections of cell from one another. Thus substrates utilized by the cell to synthesize various products as well as metabolites utilized by the cell for generating energy or growth may be separated from the synthetic and/or catabolic reactions which utilize them. With respect to product synthesis, different synthetic pathways or portions thereof, can be found in different portions of the cell. Some oxidative reactions can occur outside of the cytosol. For example, membrane bound proteins can be used to oxidize a carbon source to another intermediate.

Cystolic reactions or pathways, for example some reductions or dehydrogenations, can also be utilized to convert a substrate or intermediate into another product. When the substrate and the synthetic machinery are on opposite sides of a membrane, production of the desired end-product may require translocation of the substrate to the situs of the synthetic reaction to enable its conversion to the desired end-product . Alternatively, end-products generated Inside of the cell membrane may require translocation from within the cell. Since the partitioned sections of the cell may have different environmental parameters, e.g., solute, ion, end-product, etc., concentrations, or may require translocation across a normally impermeable barrier, some form of active transport may be required.

Responsive to these problems, investigation related to increasing the transport of materials across membranes has occurred. Solvents or lyzing agents have been used to rupture the membranes, enabling the crossing of the materials across membrane. However, such methods have adverse effects upon the viability of the host cell. If the synthetic or metabolic pathways are dependent or energized by a host cell's own metabolic or catabolic pathways, such as requiring co-factors such as NADH or NADPH, destroying the viability of the cell halts further or continued synthetic production by the host cell. In addition, while the increase in the transport of glucose or other saccharides has been explored in Increasing the growth of the cell (Parker, C., et al, Mol. Microbiol 15(5):795-802 (1995)), altering cellular transport systems to increase the industrial production of chemical end-products or intermediates through biosynthetic pathways utilizing recombinant microorganisms has not been recognized.

Cornish (J. of Gen. Microbiol., 134:3111-3122 (1988)) discusses the relationship between glucose transport and the production of a succinoglucan exopolysaccharide by *Agrobacterlyum radiobacter.* Cornish proposed that glucose uptake was a major kinetic control point for succinoglucan production, and that it ought to be possible to obtain even higher rates of succinoglucan production by using recombinant DNA methods to obtain even higher rates of succinoglucan production. However the production rates of Cornish were not on the scale of industrial needs. Furthermore, the high levels of energy expended and complex regulatory mechanism Involved In transporting glucose could discourage rather than encourage its use.

Volschenk, H., et al (Nat. Blotechnol. 15:253 (March 1997) describes the introduction of malate degradation pathways into Sacchaomyces cerevisiae by the cloning and expression of heterologous DNA encoding the same for the purpose of depleting the malate levels present in wine. Volschenk was primarily concerned with the removal of malate from the surrounding medium, not the production of any desired end product on an industrial scale.

Furthermore, the mere knowledge that a transport system is involved does little to guarantee the enhanced production of the desired end product or intermediate. The synthetic machinery may already be saturated and thus an increased presence of the substrate won't necessarily result in the Increased production of the desired end product. In addition, increased transport of a substrate that is utilized directly or indirectly as a metabolite in addition to its use as a production substrate may not result in the increased production desired.

Merely increasing the expression level of the enzyme converting the substrate to the desired end product may not result in an increased production of chemical compounds using recombinant microorganisms. There is a need for a means to increase the production of chemical compounds using recombinant microorganisms by means other than increasing the expression level of substrates within the cell.

There is also a need for a means to increase the industrial production of 2-KLG through biosynthetic pathways utilizing recombinant microorganisms by means other than increasing the expression level of the cytosolic reductases in the cell.

### SUMMARY OF THE INVENTION

The capacity of the 2,5-DKG transport of a microorganism may become a limiting factor or bottleneck to a desired 2-KLG production, in particular since 2-KLG production is compartmentalized In the cytosol and requires the transport of 2,5-DKG from its situs of production, extracellular membrane bound pathways. The present invention provides a means for alleviating that bottle neck.

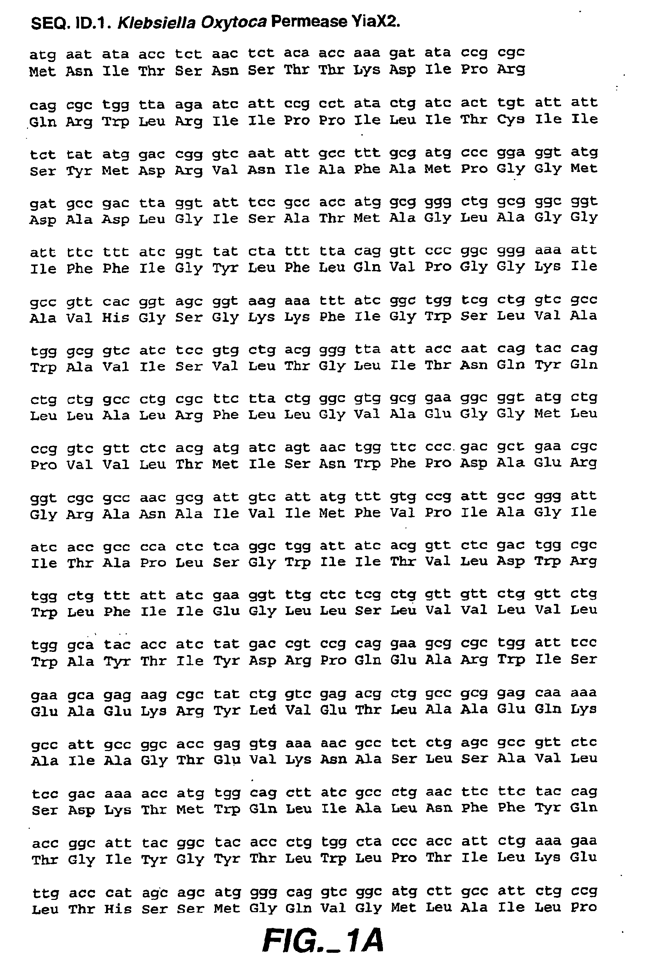

Described herein are isolated nucleic acid and amino acid sequences for P.citrea PE1, PE6, YiaX2, PermA and PermB. The amino acid sequence and nucleic acid sequence for P.citrea PE1, PE6, YiaX2, PermA and PermB is shown in FIGS. 1A-1E SEQ ID NOS: 1 and 2.

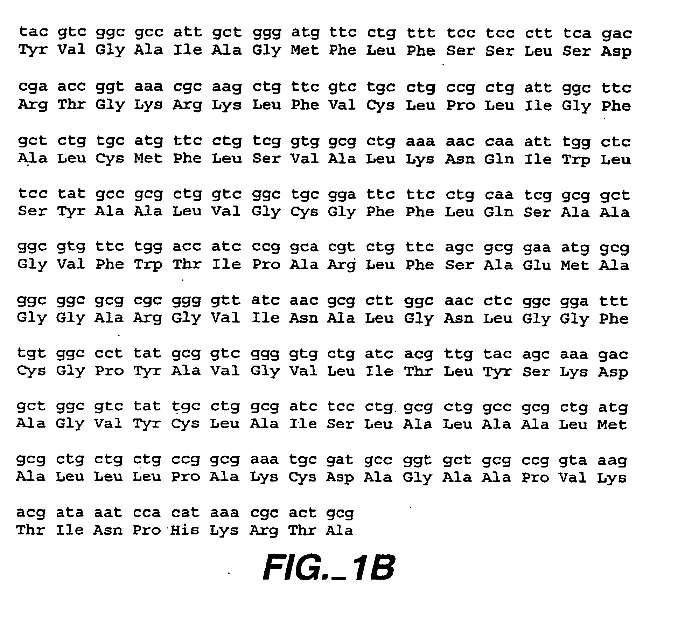

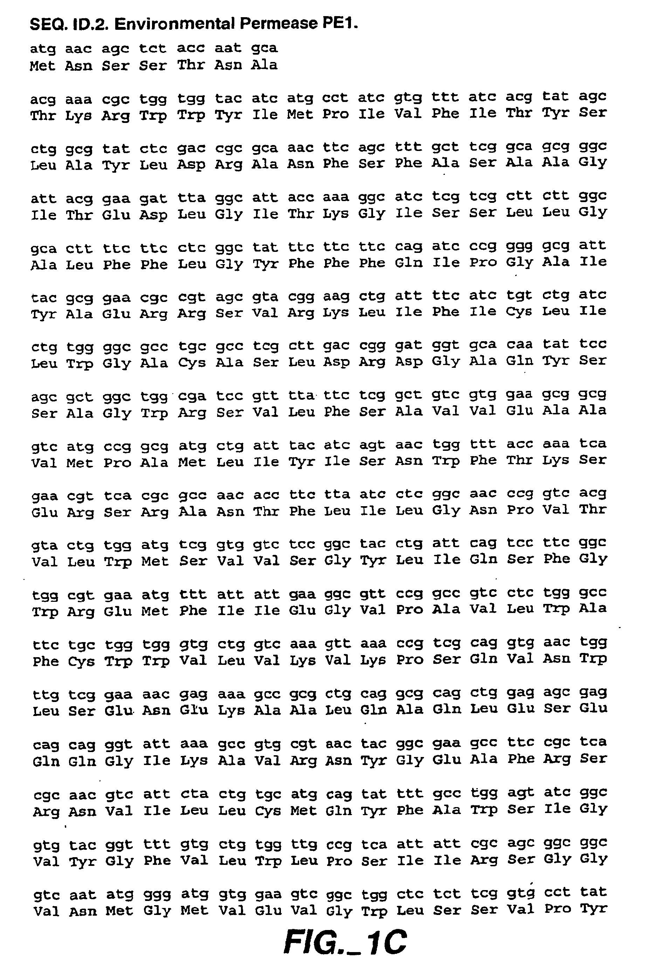

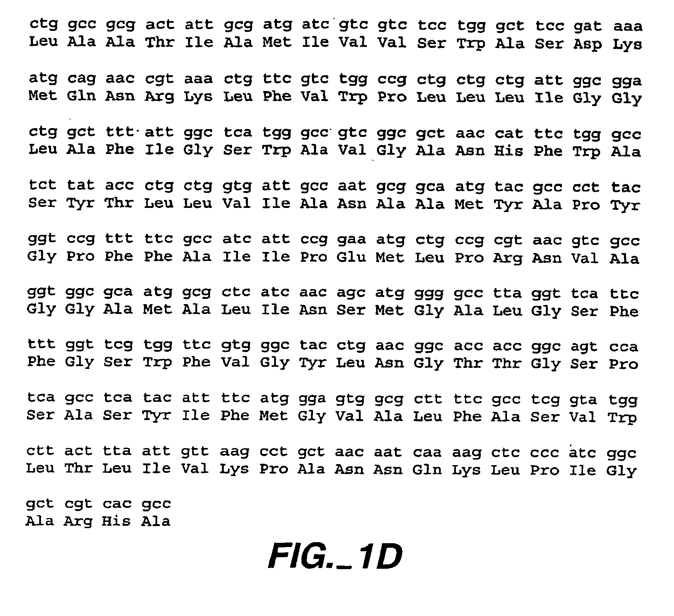

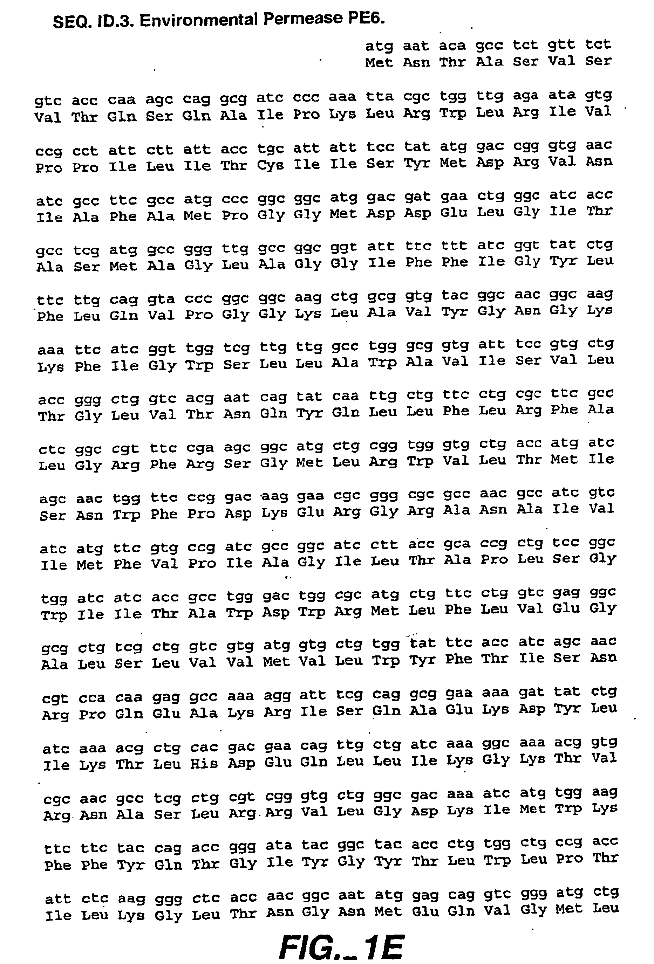

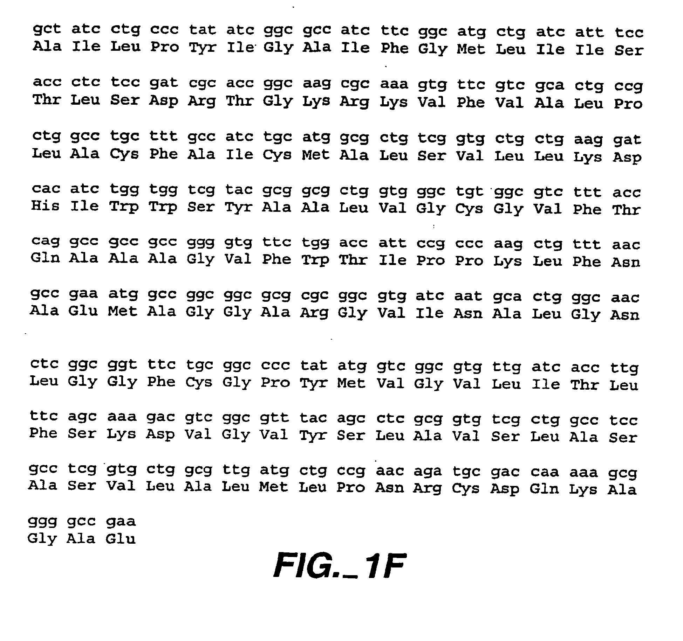

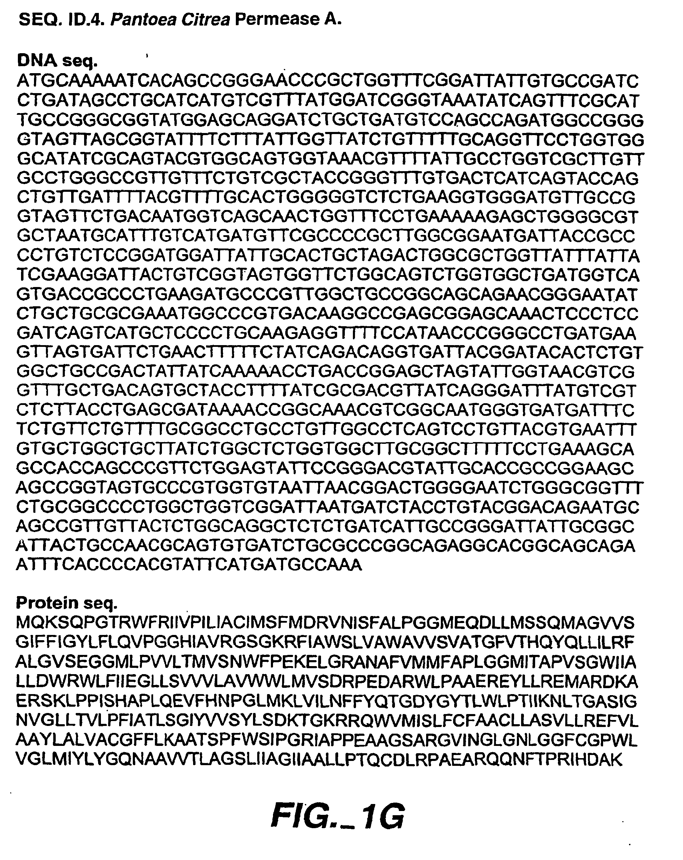

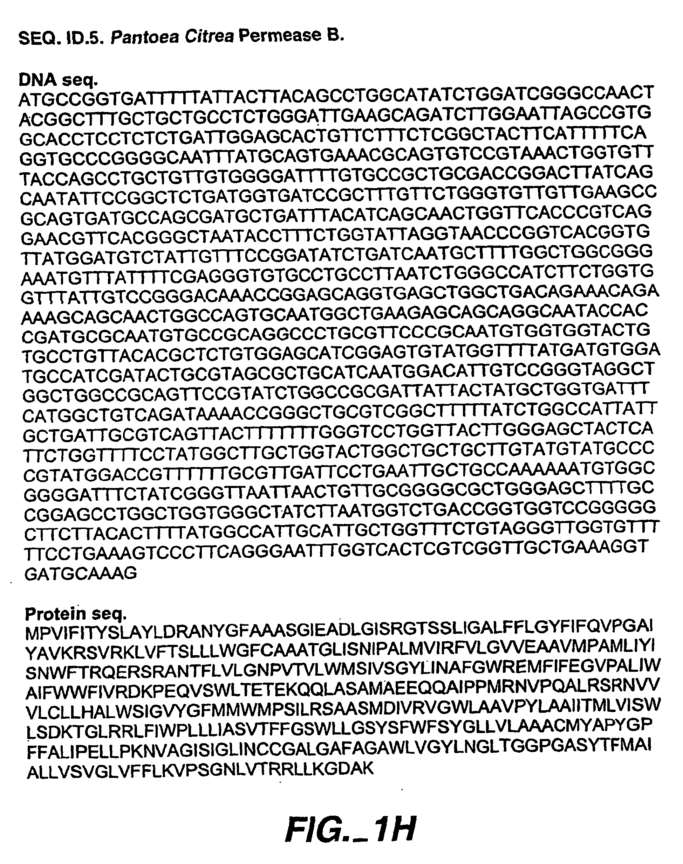

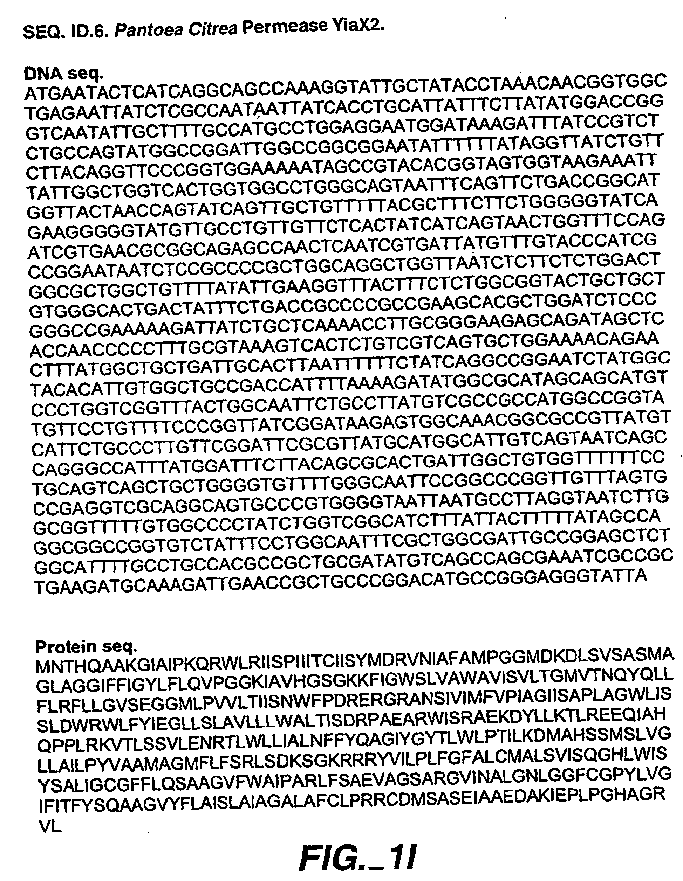

The present invention provides improved methods for enhancing a host cell's biosynthetic production of 2-KLG from 2,5-DKG. Accordingly, a method is provided for enhancing a host cell's biosynthetic production of 2-KLG, the method comprising selecting a host cell that has a synthetic pathway which converts 2,5-DKG to 2-KLG; Increasing the transport of said 2,5-DKG into said host cell while maintaining the integrity of the host cell; culturing the host cell to produce said 2-KLG; and producing the 2-KLG. In another embodiment, the step of increasing the transport of said 2,5-DKG Into said host cell includes the step of transforming into said host cell DNA encoding for one or more proteins transporting said 2,5-DKG into said host cell's cytosolic material. The said one or more proteins may be selected from the group consisting of YiaX2, PE1, PE6, PrmA and PrmB. The DNA encoding said one or more proteins may be capable of hybridizing with the appropriate sequence of Figure 1. The DNA may have at least 65% or at least 90% identity with the appropriate sequence of Figure 1.

The present invention also provides a method for enhancing the transport of 2,5 DKG into the cytosol across the inner cell membrane. the method comprising selecting a host cell; and transforming into said host cell DNA encoding for one or more proteins capable of transporting 2,5 DKG into said host cell. The one or more protein is selected from the group consisting of YiaX2, PE1, PE6, PrmA and PrmB. In one embodiment, the host cell is selected from the group consisting of bacteria and yeast. Preferably the host cell is selected from the group consisting of E. coli, Pantoea, and Klebsiella.

### BRIEF DESCRIPTION OF THE DRAWINGS

FIG. 1 shows the DNA and amino acid sequences of YiaX2 of Klebsiella oxytoca; PE1 (environmental permease);PE6 (environmental permease);PermA of Pantoea citrea; PermB of Pantoea citrea: YIaX2 of Pantoea citrea.
FIG. 2 is a flow diagram showing the synthetic pathway for the production of ascorbic acid precursor 2-KLG from glucose.
FIG. 3 is a diagram showing the synthetic pathway of the ascorbic acid precursor 2-Keto-L-gulonic acid (2-KLG).

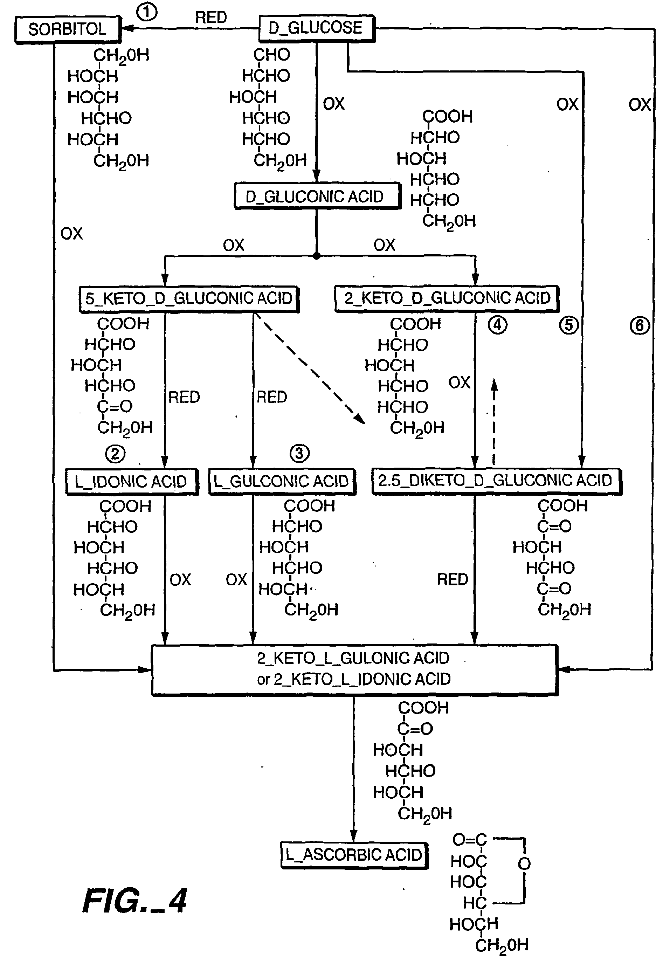

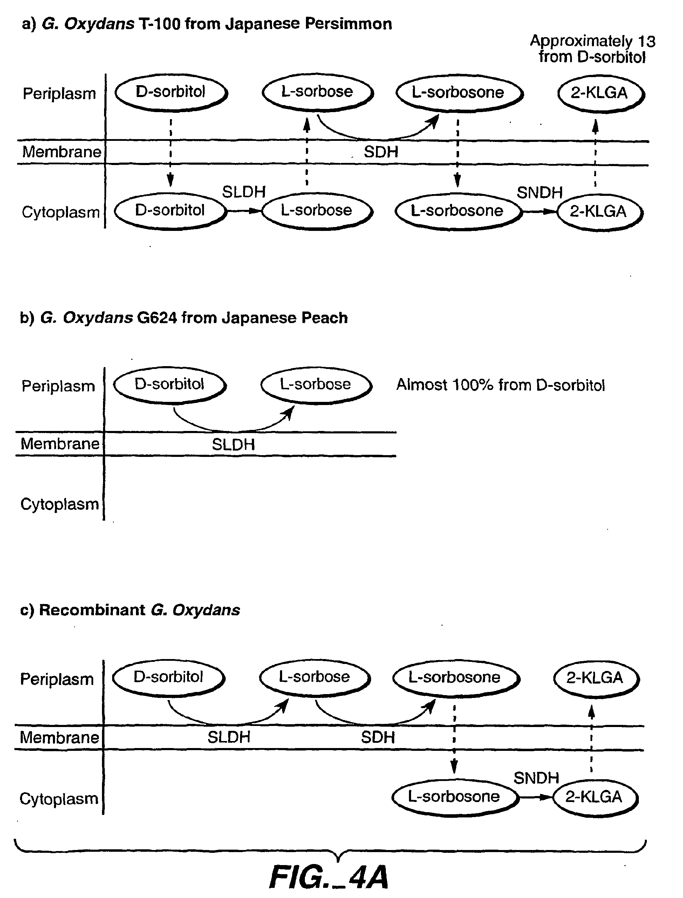

FIG. 4 is a flow chart showing the various synthetic pathways that glucose can follow to get to 2-KLG. Boudrant, J., Enzym Microb. Tech., 1990, 12, 322-329
FIG. 4A is a diagram showing the synthetic pathways of D-sorbitol to 2-KLG showing the cellular location of the reactions relative to other reactions within the pathway and the transport of substrates across cell membranes. Saito, Y, et al Biotechnol. Bioeng. 58(2/3):305-315 (1998).
FIG. 5 shows the synthetic pathway of D-glucose (G) to 2,5-DKG to 2-KLG, showing the location of the reactions relative to other reactions within the pathway and the transport of the respective substrates across the cell membrane.
FIGS. 6 is a line graph comparing the DKG transport rate with the KLG production rate showing the amount of 2,5-DKG uptake (nmoles/OD 600) v. time (-◆- = fructose feed 139-2a, 0.0486x + 0.67; - ▲- = glucose feed 139-2a, y= 0.0497 + 0,5877; -●- = seed flask 139-2a, y=0.0075x+0.0569).
FIG.7 is a schematic of the yia operon of ascorbic acid catabolism in Klebsiella oxytoca .
FIG. 8 is a graph showing the amount of 2,5 DKG uptake (nmol) by Klebsiella oxytoca measured by the silicone oil transport assay (-◆- = Tester + isopropyl β-D-Thiogalactopyranoside **[IPTG])** ; - ▲ - = ΔyiaaX2 + IPTG); - **■** - = Tester - IPTG)
FIG. 9 is a schematic drawing showing the selection design to close permeases from P. citrea, K. oxytoca and environmental sources.
FIG. 10 is a bar graph showing 2,5-DKG uptake activity in K.oxytoca strains (YiaX2, pcp1, pcp10, pcp32, pK1, Environmental #1; and Environmental #6).
FIGS. 11 is a bar graph showing 2,5-DKG uptake assay of shaker flask having various DKG permeases (139-2A, 139-2A + PCP32; 139-2A + PCP10; 139-2A + PK1; 139-2A+ PCP1) and 139-2A + PE8.in the same plasmid construct (pBCL 1920) measuring the DKG Uptake rate (g/l/hr) at 28 degrees C.9A-9B.

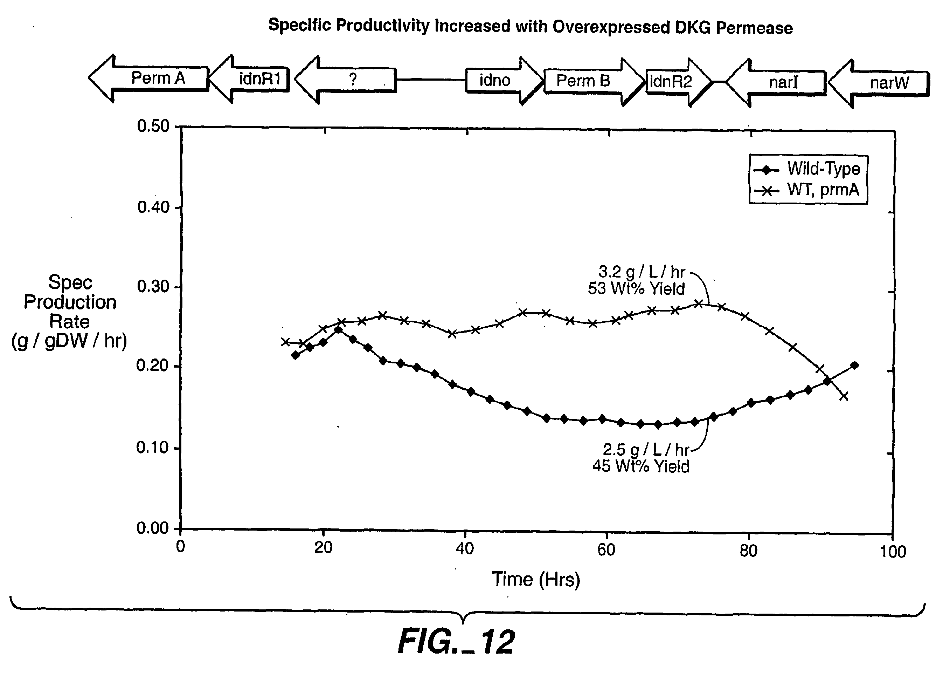

FIG. 12 is a line graph showing the specific productivity Increase with overexpressed DKG permease/ Spec Production rate (g/L/hr) (- ◆ - = wild-type; -x- = WT, prmA)
FIG. 13 is a schematic drawing of the PermA transporter in a membrane surface. The putative membrane spanning domains numbered I-XI. The positions of the conserved residues are indicated in bold. N Is the amino-terminus and C is the carboxyl-terminus. Putative membrane-spanning domains of Pantoea citrea Permease A were deduced using the tool available to http://sosui.protsome,bio.tuat.ac.jp/soculframe0.html

### DETAILED DESCRIPTION

### Definitions

### Transporter definitions

Bacterial channel transporters refers to those transporters generally in the TC classification of #1.A (Saler, M., et al., 1998, Advances In Microbial Physiology (Poole, R.K., ed.) pp. 81-136, Academic Press, San Diego, CA.). ("TC" stands for "Transport Council", a classification system which takes Into consideration the phylogenetic aspects of the transporter.) These generally transport substrates, ions or other material via an energy independent facilitated diffusion mechanism employing a transmembrane pore.

Primary transporters refers to those transporters generally In the TC classification of (TC #3.A)(Saler, M., et al, 1998), and are those that utilize chemical energy, typically in the form of ATP hydrolysis as a mode of energy coupling for the active uptake and transport extrusion of substrates.

Group translocation systems refers to transporters in the TC classification of TC #4.A. (Saier, M., et al, 1998) are transporters that concommittantly transport and phoshorylate their substrates during transport. The members of this category generally are part of the bacterial specific phosphotransferase system (PTS) and are characterized by the coupling to the oxidation of phosphoenol pyruvate (PEP) utilization.

Secondary transports refers to those transporters generally in the TC classification of #2.A,( **Saier, M., et al**, **1998**) those that generally use chemiosmotic energy, for instance in the form of a proton gradient, to provide energy to transport the substrate, ions or end products across the membrane.

Major facilitator superfamily (MFS) refers to secondary transporters that are generally in the TC classification of #2 (**Saier, M**., **et al, 1998**).

A transporter refers to any macromolecule that allows the translocating of a chemical compound across a cell membrane and into or out from a cell or cellular compartment. Transporters are also known or referred to as permeases. While not being limited to a specific theory, it is thought that the transporter is a protein that interacts with a membrane, with portions of the protein extending from the outer surface of the membrane, through the membrane, and from the inner surface of the membrane.

Active transport refers to transport that is coupled with an expenditure of energy, for example the hydrolysis of adenosine tri-phosphate (ATP) or phosphophenolpyryvate (PEP).

An anion/cation symporter refers to a transporter that utilizes an chemoisomotic gradient to transport the substrate across the membrane (TC class 14). They are also refered to as substrate/H+ symporters.

### TMS refers to transmembrane spanning domains

### Pathway definitions

Cytoplamic refers to being within the inner cell membrane.

Exogenous substrate refers to a material, found on the opposite side of the separating membrane from the synthetic reaction, e.g., outside of the inner cell membrane when the substrate is to be converted by an intracellular synthetic pathway or an intracellular portion of a synthetic pathway to the desired end product or intermediate.

Extracellular or outside the Inner cell membrane refers to cell locations on the opposite side of a membrane from the cytoplasm, including, but not limited to the periplasm.

Inner cell membrane refers to the barrier that separates the cytoplasm from the periplasm.

Membrane refers to a lipid bilayer that is intrinsically impermeable to the substrate.

Intracellular refers to the portion of the cell on the side of the membrane that is closest to or of the cytosol. Intracellular also includes cytosolic.

Intracellular reaction refers to a synthetic reaction or bioconversion located within the cytosolic cell material, i.e., material enclosed inside of the inner cell membrane.

Rate limiting step refers to the step within the 2-KLG biosynthetic pathway, where an Increase in the conversion across that step results in an increase in the production of 2-KLG.

Enhancing the production refers to increased titer (total amount) of the desired intermediate, end-product or precursor of a synthetic reaction, generally measured by an increase in the gm/l/hour obtained through the process. It may also refer to an increase in the rate at which the desired products are made, generally measured in g/l per unit time.of the recombinant production, wherein the amount of end-product, intermediate or precursor produced increases as a result of the transforming of DNA encoding the at least one protein increasing the transport of the substrate across a membrane in the presence of the overexpressed transporter.

**A substrate refers to 2,5-DKG that is bioconverted by a synthetic reaction, the cytosolic reaction situs being separated from the substrate by a membrane.**

Synthetic reaction refers to the recombinant bioconversion of a substrate to an intermediate or an end-product.

2,5-DKG reductase refers to a protein which is capable of catalyzing the conversion of 2,5-DKG stereoselectively to 2-KLG.

2,5-DKG transporter refers to a protein which is capable of transporting the 2,5-DKG across the Inner cell membrane for conversion to 2-KLG by a 2,5-KLG reductase.

### Expression definitions

Promoters refers to DNA elements that guide the RNA polymerase to start the transcription of a gene at the appropriate site to generate a messenger RNA capable of forming a polypeptide once it is translated by the translational machinery of the cell.

An "upstream activating sequence" is a binding position for a positively-acting DNA binding regulator. As indicated by its name, the upstream activating sequence is upstream of the transcription start site and is a nucleic acid.

Regulatory regions refers to regions on the DNA that modulate the expression of genes. One mechanism for this modification is that some regulatory regions serve a binding sites for proteins (also known as repressors). Once bound, a repressor interferes with the ability of RNA polymerase to transcribe a gene.

An expression system includes one or more proteins and/or nucleic acids which, when acting together, can increase the expression of a protein in a host cell. The expression system can be encoded on one or more plasmids and may or may not be on the same plasmid as the gene encoding the protein of interest.

The phrase "functionally linked" or "functionally coupled" means that the regulating elements (DNA or protein) interact physically in order to exert their function. This can be a protein/protein, DNA/protein or a DNA/DNA interaction. For example, the DNA binding regulator interacts with the promoter but genes encoding them may be at different sites on the chromosome. As such, the genes encoding the elements can be on different plasmids from each other and from the gene encoding the protein of interest and still work together to regulate expression of the protein.

Commonly, when describing proteins and the genes that encode them, the term for the gene is not capitalized and is in italics, i.e., permA. The term for the protein is generally in normal letters and the first letter is capitalized, i.e., PermA.

### Organism definitions

"Bacteria" include microorganisms of the class Schizomycetes. Bacteria can be either Gram-negative or Gram-positive. Gram-negative bacteria include members of the genera Escherichia, Hemophilus, Klebsiella. Proteus, Pseudomonas, Salmonella, Gluconobacter, Acetobacter, Yersenia, Shigella, Vibrio, Aclnetobacter, Pantoea and Serratia. Gram-positive bacteria include members of the genera Bacillus, Clostridium, Staphylococcus, Streptomyces, Lactobacillus and Lactococcus.

Gram-negative bacteria can be pantoeans which are strains that are members of the genus Pantoeas. A preferred bacterium is Pantoea citrea. Pantoea citrea is also sometimes referred to as Erwinia herblcola or Acetobacter ceremius.

The terms "isolated" or "purified" as used herein refer to a nucleic acid or amino acid that is removed from at least one component with which it is naturally associated.

### DETAILED DESCRIPTION OF THE PREFERRED EMBODIMENTS

One embodiment of the invention is directed to a method of transforming a host cell with a plasmid that includes the nucleic acid encoding the expression system. Another embodiment of the invention is directed to a method of transforming a host cell with a plasmid that includes DNA encoding for one or more proteins increasing the transport of the substrate across the membrane. A host cell is a cell into which a plasmid of the present invention can be inserted through, for example, transformation. The host cell is preferably a bacteria and more preferably in the group of Pantoea, Escherichia; Klebsiella or Bacillus.

In another embodiment, if regulating elements are incorporated, such elements of the expression system are from E. coli and B.subtilis In one embodiment, the host cell is preferably a Gram-negative bacteria. In another preferred embodiment, the host cell is a Pantoea. The same host cell can be transformed with a further plasmid that includes a nucleic acid that encodes one or more transporters. Preferably, the transporters are encoded MFS transporters, more preferably anion/cation symporters. Exemplary transporters include those encoded or expressed to *yiaX2, permA, perm B, pe6, pe1* from Pantoea citrea or Klebsiella oxytoca and heterologous sources.

The present Invention provides novel methods for enhancing a host cell's biosynthetic production of a 2-KLG, by Increasing the transport of 2,5-DKG to ameliorate the bottleneck to pathway synthesis and the production of desired end-products, in particular when the transporters are recombinantly introduced and overexpressed by the host cell.

One embodiment of the invention is directed to a method of transforming a host cell with a plasmid that includes the nucleic acid encoding an expression system. A host cell is a cell into which a plasmid of the present invention can be inserted through, for example, transformation. The host cell is preferably a bacteria. In one embodiment, the host cell is preferably a Gram-negative bacteria. In another preferred embodiment, the host cell is a Pantoea. Preferably, the host cell is Pantoea citrea and, if regulating elements are incorporated, such elements of the expression system are from Pantoea. The same host cell can be transformed with a further plasmid that includes a nucleic acid that encodes one or more transporters. Preferably, the transporters are encoded or expressed from *yiaX2, permA, perm B*, *pe6, pe1* from Pantoea citrea.

### Synthetic Reaction

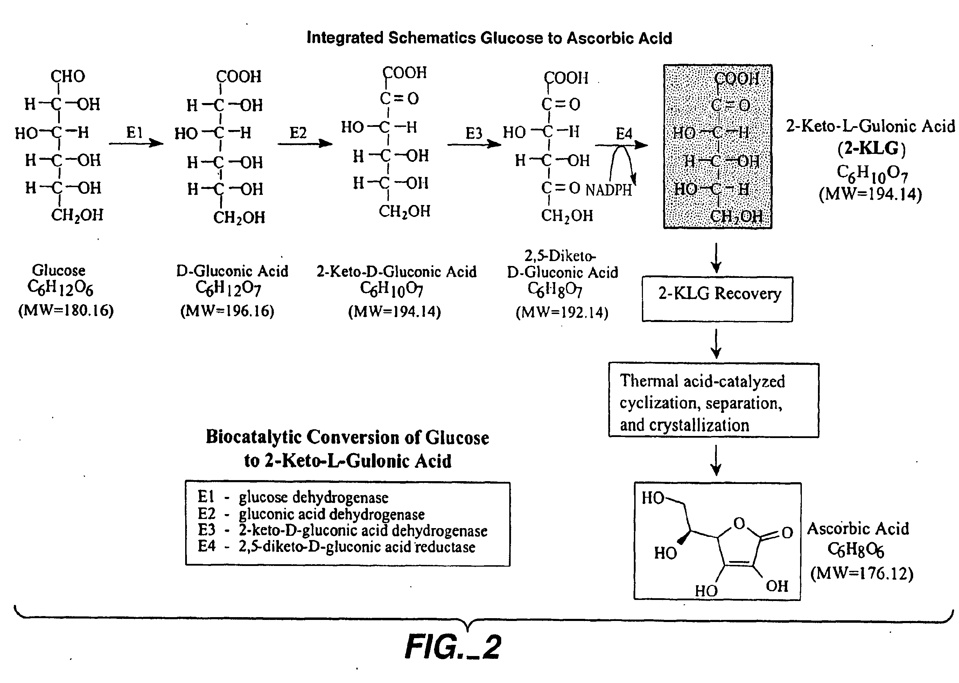

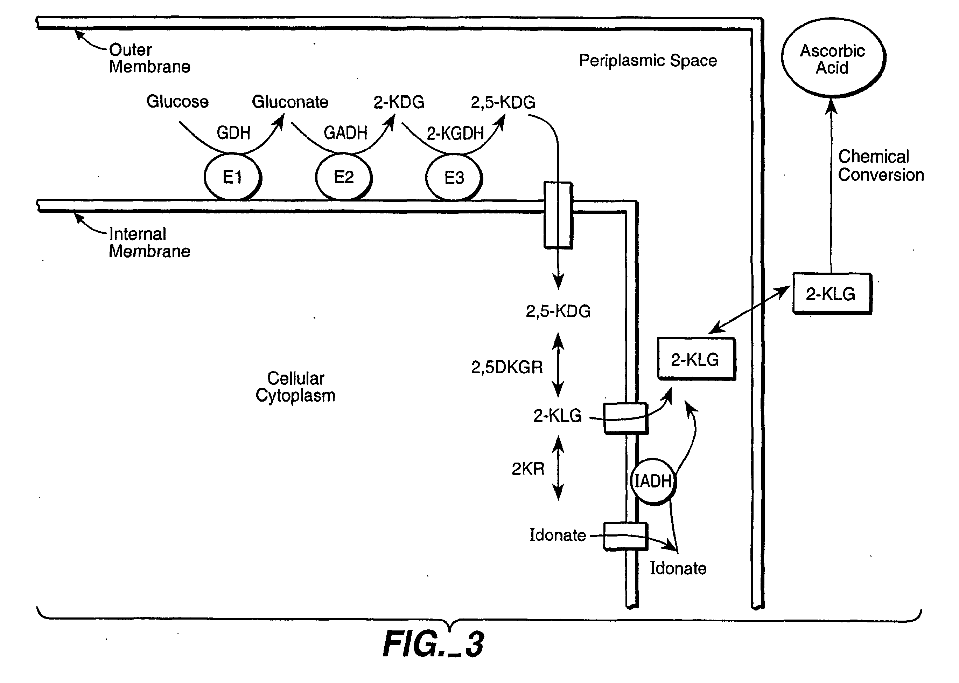

The present invention provides for the increased transport of 2,5-DKG across a membrane to enhance the production of 2,5-DKG from 2-KLG. The increased transport provides for translocation of the 2,5-DKG across a membrane separating 2,5-DKG from the cellular location of the reduction reaction (Figs. 2 and 3).

The present Invention Is particularly useful in conjunction with ascorbic acid intermediate synthesis, for example the conversion of 2,5 DKG to 2-KLG; the conversion of sorbose or sorbitol to 2-KLG via sorbosone; the reduction of 5-keto-D-Gluconic acid (5-KDG) to L-idonic acid; and the reduction of 5-Keto-D-Gluconic acid to L-gulonic acid. Each of these pathways is characterized by a portion of the synthetic pathway, a synthetic reaction, that resides within the cytoplasm, e.g. the reduction of 2,5-DKG by 2,5 DKG reductase; the reduction of L-sorbosone to 2-KLG by a sorbosone dehydrogenase; the reduction of 5-keto-D-Gluconic acid (5-KDG) to L-idonic acid by 5-KDG dehydrogenase; and the reduction of 5-Keto-D-Gluconic acid to L-gulonic acid by 5-KDG reductase. These pathways are also characterized by the necessity of transporting the substrate, e.g., 2,5-DKG; L-sorbosone, etc. across the membrane for bioconversion by the synthetic reaction residing In the cytoplasm.

The substrate is generally one that can not pass through the membrane efficiently without some sort of active transport mechanism. Preferably these can include, but are not limited to ascorbic acid intermediates (2,5-DKG, sorbosone). In addition, the substrate is a material that is transported for synthetic use on an industrial scale and generally not for metabolic use by the host cell. Industrial scale refers to the titer and volumetric productivity of being greater than 1 gm/liter/hour, preferably greater than 2 g/l/h, more preferably greater than 3 g/l/h and still more preferably greater than 5 g/l/h. In another embodiment, the productivity titer Is between 2 and 14 gm/l/hour, preferably between 3 and 12 g/l/h, and still more preferably between 5 and 10 g/l/h to be an economically viable industrial production process. Especially preferred substrates include 2,5-DKG; and sorbosone. The inventive aspects of the present invention are especially useful in these embodiments,

A reaction especially useful in the practice of this invention is the transport of 2,5-DKG across the inner cell membrane for the cystolic reduction of the same to 2-KLG by the cytosolic dehydrogenase 2,5-DKG reductase. Boudrant, J. (1990) Enzyme Microb, Technol., 1990:322-329) 2,5-DKG is converted from 2-keto-D-gluconate (2-KDG) by membrane bound 2-ketogluconate dehydrogenase. 2-KDG is converted from glucose through oxidation of D-gluconate (GA). The inventors recognize that the transport of 2,5-DKG across the inner cell membrane to the site of the cytosolic reduction to 2-KLG could be achieved by the DNA encoding an increase in the transport of 2,5-DKG.

Another reaction especially useful in the practice of this invention is the transport of sorbosone, an intermediate in the production of 2-KLG through sorbose, sorbitol (Saito, Y, et al, Biotechnol. Bloeng. 58(2/3):309-315 (19987). The conversion of sorbitol and /or sorbose to sorbosone Is a step in the pathway of converting sorbitol or sorbose to 2-KLG (**Boudrant, J., 1990; Saito (1997)).** The following is a discussion of engineering of sorbosone transporters according to the present invention.

As described Saito, the pathway of D-sorbose to 2-KLG includes the oxidation of L-sorbose to L-sorbosone by L-sorbose dehydrogenase (SDH), followed by the oxidation of L-sorbosone to 2KLG by L-sorbosone dehydrogenase. One recombinant host cell has been described which converts D-sorbitol to L-sorbosone by membrane bound dehydrogenases (Saito, Y., et al (1997)). L-sorbosone is then transported from the periplasm for reduction by L-sorbosone-dehydrogenase in the cytoplasm. Overexpression of the sorbosone transporter to facilitate the transport of the sorbosone intermediate to the pathway for conversion to 2-KLG is perceived as having a beneficial effect.

An alternative pathway of D-glucose to 2-KLG includes the oxidation of D-gluconic acid to 5-Keto-D-Gluconic acid (5KDG) which in turn is reduced to L-idonic acid (IA) or L-gulonic acid for oxidation to 2KLG. (Boudrant, J. (1990)). Transport of 5-Keto-D-Gluconic acid Into the cytosol for reduction by keto-reductase could also be facilitated by the overexpression of the 5KDG transporter.

In another embodiment, the synthetic reaction may be extracytosolic or located outside the membrane relative to the substrate. The end-product of the first reaction may be the an intermediate substrate for a second reaction on the opposite side of a membrane. For example, in the synthesis of 2-KLG from D-sorbitol in *Gluconobacter oxydans* T-100 from Japanese persimmon (FERM BP-4188), the conversion of D-sorbitol to L-sorbose by cytosolic L-sorbitol dehydrogenase results in an intermediate that is transported out of the cytoplasm, across the cell membrane for conversion to L-sorbosone by the membrane bound L-sorbose dehydrogenase. Thus the inventors contemplate increasing the transport of the cytosolic intermediate, a substrate, from the cytosolic side of the inner membrane across the membrane to outside the membrane for subsequent conversion. Saito, Y., (1997)

While a preferred embodiment includes the synthetic reaction or the pathways including the same as being within a single organism, having separate reactions in separate organisms is also contemplated by the inventors. For example, in a mixed culture system for the production of 2-KLG from glucose, the conversion of glucose to an intermediate 2,5-DKG, may occur within one organism (Acetomonas, Acetobacter, Gluconobacteer or Erwinia) while the conversion of that intermediate to the desired ascorbic acid intermediate 2-KLG occurs within the second organism (Brevibacterium, or Corynebacterium) see US Patent No. 3,963,574 to Sonoyama (1976). See also Hoshino, US Patent No. 5,312,741.

Therefore the synthetic reaction may generate an intermediate that itself may be converted at another cellular location separated by a cell membrane. The end-product, in this embodiment, may be an intermediate substrate for a subsequent reaction.

One reason for the effect of the increase transport of 2,5-DKG across the inner cell membrane to the cytosolic cell location in the production of 2-KLG is because the inventors recognized that it is the rate limiting step of the conversion of 2,5 DKG to 2-KLG. The determination of whether such a step is a rate limiting step was determined by analyzing the pathway, assessing the production of each step and altering the amount of 2,5-DKG available for conversion by the pathway to ascertain whether such increased presence of 2,5-DKG results In an increase in the overall production of the pathway. Upon determining that Increasing the presence of 2,5-DKG enhances the production of 2-KLG, increasing the transport of the substrate will enhance the production of 2-KLG.

The determination of the rate limiting step can be ascertained by comparing the productivity of the microorganism. One method for determining the rate limiting status of the pathway portion is to compare the Intermediate productivity at various points of the pathway, before and after increasing the presence of a particular chemical compound. Increasing the presence of the reductase by overexpression of the DNA encoding the 2,5-DKG reductase did not result in an increased production of 2-KLG. However, increasing the amount of 2,5-DKG present in the cytosol resulted in an Increased production of 2-KLG. Thus the inventors recognized that increasing the amount of 2,5-DKG was a rate limiting step in the production of 2-KLG.

One method for determining the rate limiting status of the pathway portion is to compare the intermediate productivity at various points of the pathway, before and after increasing the presence of a particular bioconverter. If there is no increase In the production of the end-product despite Increased presence of an intermediate or the overexpression of the converting pathway, the step may not be rate limiting, and thus overexpression of the particular enzyme effecting the synthetic reaction may not result in an enhanced production. The amounts of the individual intermediates can by determined by various indirect or direct means. Indirect means includes measuring the consumption or production of respiratory parameters, e.g. carbon dioxide production, oxygen consumption, by In-line measurements, such as gas partial pressures. Direct measurement of the intermediates can be achieved by various analytical techniques known in the artas described by Lazarus, Analyt. Biochem 157, 360-366 (1986) and references cited therein, which are incorporated by reference herein, including, but not limited to paper, gas, liquid and thln-layer chromatography as well as chemical and enzymatic assays. High performance liquid chromatography methodologies, especially as set forth in Lazarus, 1986, are especially helpful. One method used by the inventors includes the Waters 2690 HPLC and Waters 410 differential refractermeter, using a 50mM acetate buffer, 1 ml/minute flow rate, as the aluting medium and Dyonex lonpac AS-10 lon-exchange column (4x250 mm) for separating and quantifying the chemical compounds present.

Another method used by the inventors to determine the purity of the 2-kig produced in the broth was by total carbon analysis.

Thus in one embodiment, the transport activity can be measured in any cell in which the substrate can be converted to a product, by measuring production of the product in the presence of extracellular substrate. For example, in a cell naturally expressing, or recombinantly expressing, a 2,5-DKG reductase, intracellular 2,5-DKG is converted to 2-KLG. The ability of the bacterial cell to produce 2-KLG when provided with extracellular 2,5-DKG, upon expression of a 2,5-DKG permease, is a measure of the ability of the expressed permease to transport 2,5-DKG into the cell, and is thus a measure of its 2,5-DKG permease activity. Intracellular 2-KLG can be detected, for example, using HPLC or other sensitive detection methods known in the art. Other metabolic products of 2,5-DKG can also be detected, by similar methods.

### The 2,5-DKG transporter

There are four distinct types of functionally characterized transport systems based upon mode of transport and energy coupling mechanisms. The first are bacterial channel proteins (TC#1.A), which transport via an energy independent facilitated diffusion mechanism utilizing a transmembrane pore. A second transport system, the facilitators and/or secondary transporters (the second class, TC#2.A), represent the largest category of transporters. A third group ATP driven primary active transporters contsituy a use ATP hydrolysis as a mode of energy coupling for the active uptake and or extrusion of solutes. The last group consist of group transports that phosphorylate their substrates during transport (TC #4.A).

Of the secondary families, the largest of the class II families are the major facilitator superfamily (MFS) and the amino acid polyamine choline (APC) family (reizer et al. These secondary transporter families can be further divided into three groups (1) proton motive form (pmf driven), (2) sodium motive force (smf)-driven, and (3) other ion or solute driven exchangers. These transport systems catalyze uni, anti and/or symport of solutes. Secondary active transporters have been identified in E.coli, H.influenzae, H. pylori, B. subtilis, M. genitalium, Synechocystis, M.Jannaschii (Paulsen et al 1998 J. Mol. Biol. 277:573-592). All members of this category are part of the bacterial specific phosphotransferase system (PTS). Secondary transporters are typically polytopic membrane proteins, frequently with 12 TMS with most primary carriers, a chemical form of energy drive the group translocation, be it ATP-dependent systems as most ATP-binding cassette (ABC) superfamily members are, or PTS, which use PEP as the phosphyoryl donor for sugar uptake and phosphorylation. Secondary transporters differ from primary (ABC transporters) in that the primary transporters use ATP, taking energy away from the cell. In addition, the use of ABC transporters requires a more complex transporter system, one that comprises two hydrophobic integral membrane domains, and two ATP-binding domains (Hosie, et al Molecul. Microbiol (2001) 40(6), 1449-1459. Those ABC transportes responsible for the uptake of solutes also require the presence of a solute-binding protein (SBP). Thus genetic engineering of an Improved ABC transport system would require the expression and transformation of a more complex nature than one of a secondary transporter.
In one embodiment, the DNA encoding the at least one protein for increasing the transport of the substrate across the inner cell membrane is selected from Acetobacter, Pseudomonas, Bacterium, Cyanococcus, Micrococcus, Brevibacterium, Arthrobacter, Staphylococcus, Bacillus, Corynebacterium, Acetomonas, Gluconobacter and Erwinia. Prefered organisms are selected from the group consisting of E.coli, Pantoea and Kleibsiella. Pantoea Is the most preferred organism to use as a host cell.

Especially useful transporters include those encoded by yiaX2 (from Klebsiella oxytoca), pe1 and pe6 (from environmental sources), and yiaX2, permA and permB from Pantoea citrea. yiaX2, permA and permB genes can be found in a variety of bacteria such as Erwinia, Acetobactor, Gluconobactor, E. coli, Agrobactor, Yersenia, Salmonella, Corynebacterium, Brevibacterium, Arthrobacter, Micrococcus, Staphylococcus, Pseudomona, Bacillus, Citrobacter. Species include as Yersenia pestis, Yersenia pseudotuberculosis, Salmonella typhimurium, Pseudomonas aeruginosa, Streptomyces coelicolor.

Figure 1 shows nucleic acid sequences of DNA encoding YiaX2 , PermA, PermB PE1 and PE6 which may be used as DNA encoding the at least one enzyme increasing the transport of the substrate across the membrane in the host cell.

The present invention encompasses the use of YiaX2, PermA, PermB, PE1 and PE6 polynucleotide homologs encoding transporters YiaX2, PermA, PermB, PE1 and PE6 , respectively, whether encoded by one or multiple polynucleotides which have at least 65&, 70%, 80%, or at least 90% or at least 95% identity to P. citrea YiaX2, PermA, PermB, PE1 and PE6, respectively as long as the homolog encodes a protein that is able to function by modulating transport, preferably increasing transport, of a substrate in a microorganism. As will be understood by the skilled artisan, due to the degeneracy of the genetic code, a variety of polynucleotides, i.e., YiaX2, PermA, PermB, PE1 and PE6 polynucleotide variants, can encode the Pantoea citrea transporters on factors YiaX2, PermA, PermB, PE1 and PE6. The present invention encompasses use of all such polynucleotides.

Microorganism polynucleotide homologs of P. citrea, Klebsiella oxytoca and environmental isolates YiaX2, PermA, PermB, PE1 and PE6 transporters can be identified through nucleic acid hybridization of microorganism nucleic acid of either genomic of cDNA origin. The polynucleotide homolog sequence can be detected by DNA-DNA or DNA-RNA hybridization or amplification using probes, portions or fragments of the DNA encoding the at least one polynucleotides transporting the 2,5-DKG into the host cells cytosolic material.

Also included within the scope of the present invention is the use of YiaX2, PermA, PermB, PE1 and PE6 polynucleotide sequences that are capable of hybridizing to part or all of the YiaX2, PermA, PermB, PE1 and PE6 nucleotide sequence of FIGS. 1 under conditions of intermediate to maximal stringency. Hybridization conditions are based on the melting temperature (Tm) of the nucleic acid binding complex, as taught in Berger and Kimmel (1987, Guide to Molecular Cloning Techniques, Methods in Enzymology, Vol 152, Academic Press, San Diego CALIF.) incorporated herein by reference, and confer a defined "stringency" as explained below.

"Maximum stringency" typically occurs at about Tm-5.degree. C. (5.degree. C. below the Tm of the probe); "high stringency" at about 5.degree. C. to 10.degree. C. below Tm; "intermediate stringency" at about 10.degree. C. to 20.degree. C. below Tm; and "low stringency" at about 20.degree. C. to 25.degree. C. below Tm. As will be understood by those of skill in the art, a maximum stringency hybridization can be used to identify or detect identical polynucleotide sequences while an intermediate or low stringency hybridization can be used to identify or detect polynucleotide sequence homologs.

The term "hybridization" as used herein shall include "the process by which a strand of nucleic acid joins with a complementary strand through base pairing" (Coombs J (1994) Dictionary of Biotechnology, Stockton Press, New York N.Y.).

The process of amplification as carried out in polymerase chain reaction (PCR) technologies is described In Dieffenbach C W and G S Dveksler (1995, PCR Primer, a Laboratory Manual, Cold Spring Harbor Press. Plainview N.Y.). A nucleic acid sequence of at least about 10 nucleotides and as many as about 60 nucleotides from the PermA nucleotide sequence of FIGS. 1A-1E, preferably about 12 to 30 nucleotides, and more preferably about 20-25 nucleotides can be used as a probe or PCR primer.

### Amino Acid Sequences

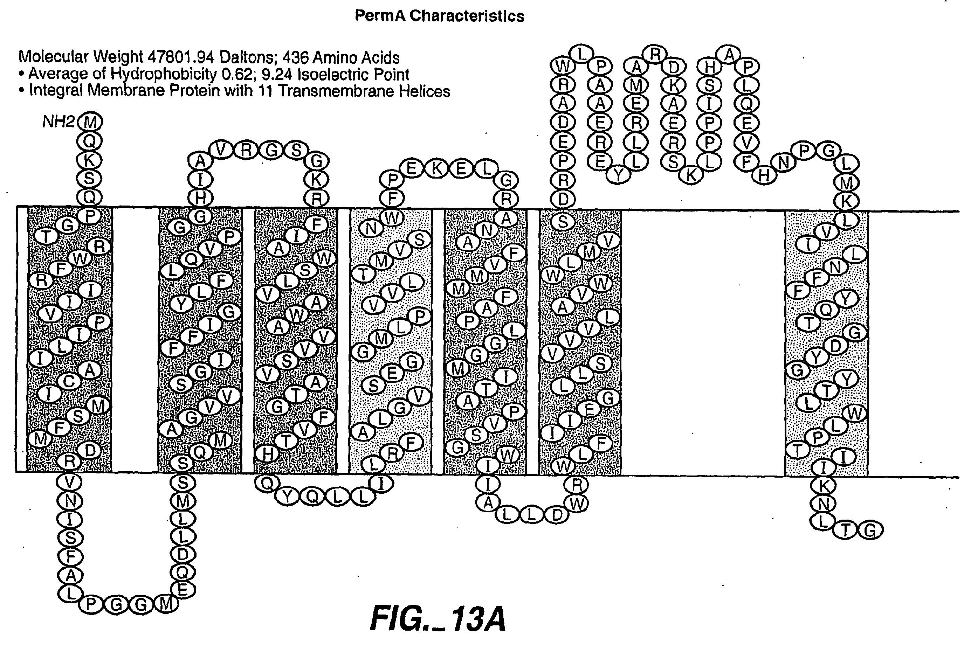

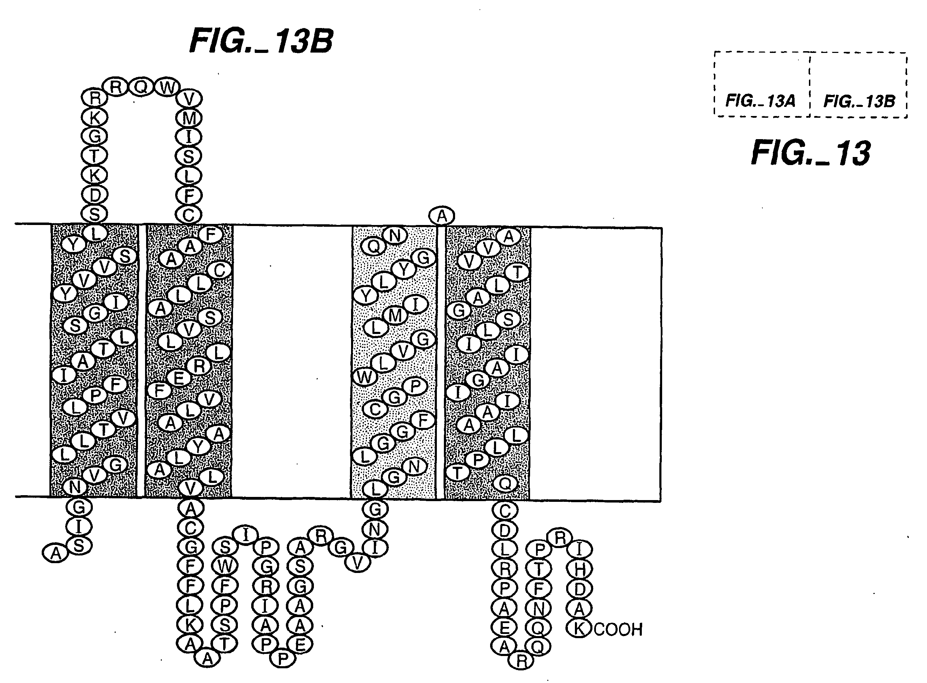

The P. citrea PermA polynucleotide as shown in Fig 1 encodes P. citrea PermA. The P. citrea permA gene specifies one protein of 436 residues with a celculated molecular mass of 47801.94 Daltons. The average hydrophobicity was 0.62 and the isoelectric point was 9.24. The permA protein is an integral membrane protein with 11 putative transmembrane helices. These domains show significant sequence similarity to other known tranporter proteins from other organisms, the highest simllarity being found with KDG transporter proteins from the Pseudomonas. Fig 13 shows the putative membrane-spanning domains (I-XI) of Perm A.

Transporters suitable for use in the present invention include those encoded by *yiaX2*, *permA, pe1, pe6*, and *permB* from Pantoea citrea, Klebsiella oxytoca and environmental sources. *YiaX2, permA, pe1, pe6, and permB* genes can be found in a variety of species of bacteria such as Erwinla, acetobacter, gluconobacter, E. coli, Agrobacter, Yersinia, Samonella, Corynebactertum, Brovibacterium, Arthrobacter, Micrococcus, Staphylococcus, Pseudomonas and Bacillus.

### II. Expression Systems

The present invention utilises expression systems for the enhanced production and transport of desired heterologous or homologous proteins in microorganisms, including bacteria and yeast.

### a. Coding Sequences

A vector for expression of a transporter may comprise at least one copy of nucleic acid encoding a transporter and preferably comprises multiple copies. In a preferred embodiment, the microorganism is Pantoea. In another preferred embodiment, the microorganism is Klebsiela. In one embodiment, polynucleotides which comprise the permA gene are utilized to construct the vector. These polynucleotide segments can comprise of a greater number of residues than permA. For example, pcp1, pcp10and pcp32 are nucleotide fragments that are operons or domains of the Pantoea citrea genome. These polynucleotide segments are about 9 kilobses (kb), 13 kb and 67 kb respectively. In a preferred embodiment, polynucleotides which encode P. citrea PermA. YiaX2, PermB, PE1, and/or PE6, or fragments thereof, or fusion proteins or polynucleotide homolog sequences that encode amino acid variants of PermA. YiaX2, PermB, PE1, and/or PE6, may be used to generate recombinant DNA molecules that direct the expression of PermA. YiaX2. PermB, PE1, and/or PE6, or amino acid variants thereof, respectively, in gram-positive host cells. In one embodiment, the host cell is selected from the group consisting of Acetobacter, Pseudomonas, Bacterium, Cyanococcus, Micrococcus, Brevlbacterium, Arthrobacter, Staphylococcus, Bacillus, Corynebacterium, Acetomonas and Gluconobacter. Prefered host cells are selected from the group consisting of Escherichia, Pantoea and Kleibsiella. Pantoea citrea and Kleibsiella are the most preferred is organisms to use as a host cell. Pantoea is also known as Erwinia.

As will be understood by those of skill in the art, it may be advantageous to produce polynucleotide sequences possessing non-naturally occurring codons. Codons preferred by a particular host cell **(Murray E et al (1989) Nuc Acids Res 17:477-508)** can be selected, for example, to increase the rate of expression or to produce recombinant RNA transcripts having desirable properties, such as a longer half-life, than transcripts produced from naturally occurring sequence.

Altered PermA. YiaX2, PermB, PE1, and/or PE6 polynucleotide sequences which may be used in accordance with the invention include deletions, insertions or substitutions of different nucleotide residues resulting in a polynucleotide that encodes the same or a functionally equivalent PermA. YiaX2, PermB, PE1, and/or PE6 homolog, respectively. As used herein a "deletion" is defined as a change in either nucleotide or amino acid sequence in which one or more nucleotides or amino acid residues, respectively, are absent.

As used herein an "insertion" or "addition" is that change in a nucleotide or amino acid sequence which has resulted in the addition of one or more nucleotides or amino acid residues, respectively, as compared to the naturally occurring gram positive PermA. YiaX2, PermB, PE1, and/or PE6.

As used herein "substitution" results from the replacement of one or more nucleotides or amino acids by different nucleotides or amino acids, respectively.

The encoded protein may also show deletions, insertions or substitutions of amino acid residues which produce a silent change and result in a functionally equivalent gram-positive PermA. YiaX2, PermB, PE1, and/or PE6 variant. Deliberate amino acid substitutions may be made on the basis of similarity in polarity, charge, solubility, hydrophobicity, hydrophilicity, and/or the amphipathic nature of the residues as long as the variant retains the ability to modulate transport, preferably to increase transport. For example, negatively charged amino acids include aspartic acid and glutamic acid; positively charged amino acids include lysine and arginine; and amino acids with uncharged polar head groups having similar hydrophilicity values include leucine, isoleucine, valine; glycine, alanine; asparagine, glutamine; serine, threonine, phenylalanine, and tyrosine.

The PermA. YiaX2, PermB, PE1, and/or PE6 polynucleotides of the present Invention may be engineered In order to modify the cloning, processing and/or expression of the gene product. For example, mutations may be introduced using techniques which are well known in the art, eg, site-directed mutagenesis to insert new restriction sites to change codon preference, for example.

In one embodiment of the present invention, a PermA, YiaX2, PermB, PE1, and/or PE6 polynucleotide may be ligated to a heterologous sequence to encode a fusion protein. A fusion protein may also be engineered to contain a cleavage site located between the PermA. YlaX2, PermB, PE1, and/or PE6 nucleotide sequence and the heterologous protein sequence, so that the PermA. YlaX2, PermB, PE1, and/or PE6 protein may be cleaved and purified away from the heterologous molety.

### b. Vector Sequences

Expression vectors used in expressing transporters in microorganisms comprise at least one promoter associated with a transporter factor selected from the group consisting of PermA. YiaX2, PermB, PE1, and/or PE6, which promoter is functional in the host cell. In one embodiment of the present invention, the promoter is the wild-type promoter for the selected transporter and in another embodiment of the present invention, the promoter is heterologous to the transporter, but still functional in the host cell.

Additional promoters associated with heterologous nucleic acid encoding desired proteins or polypeptides may be introduced via recombinant DNA techniques. In one embodiment of the present invention, the host cell is capable of overexpressing a heterologous protein or polypeptide and nucleic acid encoding one or more transporter(s) is(are) recombinantly introduced. In one preferred embodiment of the present invention, nucleic acids encoding the at least one protein or more proteins increasing the transport of the substrate maybe stably integrated into the microorganism genome. In another embodiment, the host cell is engineered to overexpress DNA encoding for one or more proteins increasing the transport of said substrate into said host cell of the present invention and nucleic acids encoding the heterologous protein or polypeptide is introduced via recombinant DNA techniques. The host cells may be capable of overexpressing other transporters known to those of skill in the art.

In a preferred embodiment, the expression vector contains a multiple cloning site cassette which preferably comprises at least one restriction endonuclease site unique to the vector, to facilltate ease of nucleic acid manipulation. In a preferred embodiment, the vector also comprises one or more selectable markers. As used herein, the term selectable marker refers to a gene capable of expression In the gram-positive host which allows for ease of selection of those hosts containing the vector. Examples of such selectable markers include but are not limited to antibiotics, such as, erythromycin, aspectinomycin, chloramphenicol and tetracycline. Also provided are embodiments utilising a transporter encoded by a nucleic acid having at least 90% homology with one of the DNA sequences shown in Figure 1. Preferably, the homology is at least 95%, more preferably at least 98%. Homology can be determined by lining up the claimed amino acid or DNA sequence with another sequence and determining how many of the amino acids or nucleotides match up as a percentage of the total. Homology can also be determined using one of the sequence analysis software programs that are commercially available, for example, the TFastA Data Searching Program available in the Sequence Analysis Software Package Version 6.0 (Genetic Computer Group, University of Wisconsin Biotechnology Center, Madison, Wis. 53705).

One can screen for homologous sequences using hybridization as described herein or using PCR with degenerate primers. Chen and Suttle (1995) Biotechniques 18(4):609-610, 612.

Also, in several embodiments of the invention, nucleic acids may be used that can hybridize with the DNA or fragments thereof, shown in Figure 1 under stringent conditions. Stringent hybridization conditions include stringent hybridization and washing conditions as is known to one of ordinary skill in the art. Hybridization and appropriate stringent conditions are described in Sambrook et al. 1989 Molecular Cloning 2d ed., Cold Spring Harbor Laboratory Press, New York.

Methods for increasing Production of Recombinant Peptides from Host Cells having transformed DNA encoding transport proteins

A particularly powerful method of increasing the transport of the substrate from one cellular location to another involves the deletion of genes resulting in metabolic diversions from the transformed host cell and the concomitant provision of DNA encoding which increases the transport of the desired substrate. This is advantageously achieved by providing to the cell a deletion-transporter chimera or fusion protein, in which the metabolic diversions of the deleted portion are minimized, and in which the transporter ability portion is overexpressed. Chemically-fused polypeptides, or shuffled sections of the genenome are a possibility, but recombinant proteins are naturally most preferred for use in this manner. The identification of appropriate permease fragments for use in such a chimera has been described herein above.

In terms of the transport portions of these fusion proteins, any permease-derived sequence that contains enough primary sequence information to confer transport activity to the chimera will be useful in this context. However, it will often be preferred to use the entire transporter enzyme as this is more straightforward in terms of methodology. Again, one may look to the extensive information available in various published references in order to assist with the identification of appropriate transporters or fragments thereof.

### Transformation

The present invention also contemplates augmenting or increasing the capabilities of cells to produce biologically active polypeptides, such polypeptides increasing the transport of a substrate from a first location of the cell across a membrane to a second location of the cell. This can be accomplished, in some instances, by overexpressing the proteins involved in the transport of a substrate to another cellular location for additional bioconversion, such as a secondary transporter of the anion/cation symporter (Saier, 1988), in one embodiment an anion/cation H+ symporter. Exemplary symporters that are contemplated by the inventors include permeases YiaX2, PE1, PE6, prmA and prmB from Klebsiella oxytoca and Pantoea citrea.

Expression of transporters involved in maintaining the viability and productive qualities of host cells, especially their transport capacity, is important. In the event that the pathways are NADPH or NADH requiring reactions, the continued viability of the host cells is a necessity for successful continuous production by the desired pathway. Certain considerations and factors can be kept in mind while determining the number of multiple copies or promoters that can be overexpressed while maintaining the viability of the organism. In general, excessive expression of transporters can be detrimental to the cells because the available space to incorporate the transporter in the membrane is limited. Therefore, very excessive overproduction of one transporter may decrease the incorporation in the membrane of other transporters that may be involved in the transport of other nutrients from the media . Engineering the overexpression of a cell type-specific transcription factor could increase or stabilize the transporter capabilities of engineered host cells. Stable overexpression of H+ symporters in bacterial host cells will serve several purposes. It will increase transgene expression under while maintaining the viability of the microorganism. The overexpression of the symporters is simpler than overexpression of ABC transporters since symporters do not require the extensive encoding for the multiple components of the ABC transporter. Thirdly, by overexpressing a secondary transporter Instead of ATP or PTS coupled transports diversion of energy requirements from the host cell is minimized.

In one embodiment of the present invention, nucleic acid encoding one or more transporters is introduced into a host cell via an expression vector capable of replicating within the host cell. Suitable replicating plasmids for Pantoea are described in Sambrook, et al, 1989, Molecular Cloning 2d ed., Cold Spring Harbor Laboratory Press, New York), based on the fact that Pantoea sustain the replication of the same plasmids that E.coli.

In another embodiment, nucleic acids encoding one or more trasporters is stably integrated into the microorganism genome. Preferred host cells are from the genus Pantoea. Another preferred host cell is K. oxytoca. Several strategies have been described in the literature for the integration of DNA into the chromosome (see for example Balbas, et al., 1996, Gene 172:65-69; LeBorge, et al, 1998, Gene 223: 213-219)

Transformation of P.citrea can be accomplished by the electroporation method, using the protocol developed for E.coli (Potter, H., 1988, Anal.Biochem. 174:361-373).

### III. identification of Transformants

After introducing the DNA into the host, the transformants are selected by antibiotic resistance encoded in the vector or in general by selecting for a function coded within the plasmid. Once transformants have been differentiates form non-transformed cells, the presence of the plasmid with an Intact structure can be confirmed using standard protocols (Sambrook, et al, 1989)

The presence of the YiaX2, PermA, PermB, PE1, and Pe6 polynucleotide sequence can be detected by DNA-DNA or DNA-RNA hybridization or amplification using probes, portions or fragments of the polynucleotide sequence as disclosed in FIG 1.

### IV. Transport Assays

A preferred method for determining the levels of the ascorbic acid intermediates by HPLC methodology has been discussed supra.

In additon, a wide variety of labels and conjugation techniques are known by those skilled in the art and can be used in various nucleic and amino acid assays. Means for producing labeled hybridization or PCR probes for detecting specific polynucleotide sequences include oligolabeling, nick translation, end-labeling or PCR amplification using a labeled nucleotide. Alternatively, the nucleotide sequence, or any portion of it, may be cloned into a vector for the production of an mRNA probe. Such vectors are known in the art, are commercially avallable, and may be used to synthesize RNA probes in vitro by addition of an appropriate RNA polymerase such as T7, T3 or SP6 and labeled nucleotides.

A number of companies such as Pharmacla Blotech (Piscataway N.J.), Promega (Madison Wis.), and US Biochemical Corp (Cleveland Ohio) supply commercial kits and protocols for these procedures. Suitable reporter molecules or labels Include those radionudides, enzymes, fluorescent, chemiluminescent, or chromogenic agents as well as substrates, cofactors, Inhibitors, magnetic particles and the like. Patents teaching the use of such labels include U.S. PaL Nos. 3,817,837; 3,850.752; 3,939,350; 3,996,345: 4,277,437; 4,275,149 and 4,366,241. Also, recombinant immunoglobulins may be produced as shown in U.S. Pat. No. 4,816,567. Also, recombinant immunoglobulins may be produced as shown in U.S.Pat. No. 4,816,567.

### EXAMPLES

### Example 1

### Materials and Methods

a. Plasmid, bacterial strain and media: Plasmid pBCL1920, K. oxytoca, P. citrea 1392A. P. citrea 1392A strain is a P. citrea variant which is 39140. pD92 Is described in stands for a vector which contains DKG reductase gene. U.S. Patent No. 5,376,544 . Murphy III medium contained fructose 0.5%, Phosphate 1.6%, MgSO4.7H2O 0.2%, Soytone 0.2%, citrate 0.01%, (NH4)2SO4 1%, Trace salts In ppm range such as Fe, Co, Mn, Zn and vitamins such as nicotinic acid, folate and B12 ; M9 medium, 0.9% Phosphate, 0.1% NaCl, 0.1% NH4Cl, MgSO4 0.0005%, CaCl2 0.025%; Fermentation medium Potassium. Phosphate 1%, MgSO4 0.15%, glutamate 1.5%, fructose 2.5%, ammonium sulfate 0.1%, and vitamin blend having biotin, thiamin, pyridoxine, riboflavin, nicotinic acid, folic acid and B12 ,and trace metals cocktail solution having iron, Zn, and Mn ions in 10-100 ppm level; Transport assay medium 100 mM Potassium Phosphate pH 6.9 ; Antibiotics Spectinomycin 50 ug/ml and Tetracyclin 20 ug/ml, IPTG 100 uM.

### b. DNA techniques

c. Growth of cells : Strains constructed using recombinant methods and wild-type P. citrea and K. oxytoca were grown in either M9 medium containing DKG as the sole carbon source or MIII medium with fructose as the sole carbon source or with MIII having mixed carbon source such as fructose, Gluconate and DKG in the range from 0.1 % to 1%. Cells were grown between 20-37°C but preferably below 30°C. Cells were preferably grown at neutral pH but the range comprised of from pH 5-8. P. citrea cells grown in a fermenter from a seed flask used fermentation medium using either fructose or glucose feed.

d. DKG uptake biochemical assay: Samples of fermentation broth containing cells were withdrawn from respective growth apparatus and were quenched on ice-water bath. The fermentation broth was centrifuged and supernatant was discarded. The cell pellet was washed using 0.95 ice-cold saline solution followed by 2 washes by DKG uptake assay buffer 100 mM ice-cold Potassium phosphate pH 6.9. Cells were resuspended in the same assay buffer to an OD of 12 at 550 nm and were incubated at room temperature or preferably at 28°C. DKG uptake assay was started by mixing the cells with C-14 enriched radio-isotoped 2,-5 DKG. Time course of DKG uptake was performed using vaccum/filter based quenching using ice-cold assay buffer. DKG uptake measurement were done by radioisotope incorporation in the cells and the data obtained was plotted against time to give the DKG uptake rate.
Another assay using silicone oil for cellular DKG uptake and metabolism study was also performed (Johnson, J.D. et al., J. Lab. Clin. Med., 1980, 95: 429-439). 100ul of silicon oil was added to epitubes ready for the assay. Cells and the 14C (U) enriched 2,5-DKG was mixed and then at a regular time intervals 100ul of the cell/substrate mixture was withdrawn and added to the epitubes containing silicon oil, centrifuged for 15 seconds and then immediately frozen in dryice/ethanol bath. After five minutes, tip of the epitube containing cell pellet is cut directly into a scintillation vial. Rest of the tube is recut just above the frozen portion and dropped into another vial containing scintillation fluid. Counts were measured after after an overnight to facilitate cell pallet loosening from the epi tip. The difference between the loss of counts from top and appearance of counts in the pallet is due to the cell metabolism and released CO₂. At the end of the uptake assay, when cells were permeabilized, the low molecular cell contents leaked out and provided information about the accumulated imported substrate and fate of substrate futher metabolized to become cellular component.

e. DKG reductases assay: Cell pellets from each fermentor were collected and frozen at - 70°C for approx. 24 hours. Pellets at the 15 and 25 hour time points were thawed on ice and French pressed in 50 mM PIPES buffer, pH 6.5. The extracts were spun for 2 min. X 14 K rpm on a bench-top centrifuge, and subsequently measured for total protein concentration and reductase activity. All samples were measured by Bradford and BCA assays for protein, and diluted as needed for accurate rate assays. The assays were measured against background rates which contained all but the 2,5-DKG (and were all less than 10% of the total rates). The buffer contained: 50 mM PIPES pH 6.5, 150 uM NADPH and 5 mM 2,5-DKG.

Another assay using silicon oil for cellular DKG uptake and metabolism study was also performed (Johnson, J.D., et al., J. Lab. Clin. Med. 1980, 95:429-439). 100 ul of silicon oil was added to epitube ready for the assay. Cells and 14C (U) enriched 2,5-DKG was mixed and then at a regular time intervals (for example about every 10 seconds) 100ul of the cell/substrate mixture was withdrawn and added to the epitubes containing silicon oil, centrifuged for 15 minutes and then immediately frozen in dry ice/ethanol bath. After 5 minutes, the tip of the epitube containing the cell pellet is cut directly into a scintillation vial. The rest of the tube is recut just above the frozen portion and dropped into another vial containing the scintillation fluid. Counts were measured after an overnight to facilitate cell pallet loosening from the epi tip. The difference between the loss of counts from top and appearance of counts in the pallet is due to the cell metabolism and released CO₂. At the end of the uptake assay, when cells were permeabilized, the low molecular cell contents leaked out and provided information about the accumulated imported substrate and fate of substrate further metabolized to become cellular components.

### Example II

Example II provides the basis that transporters of substrate may be rate limiting in a whole-cell bioconversion

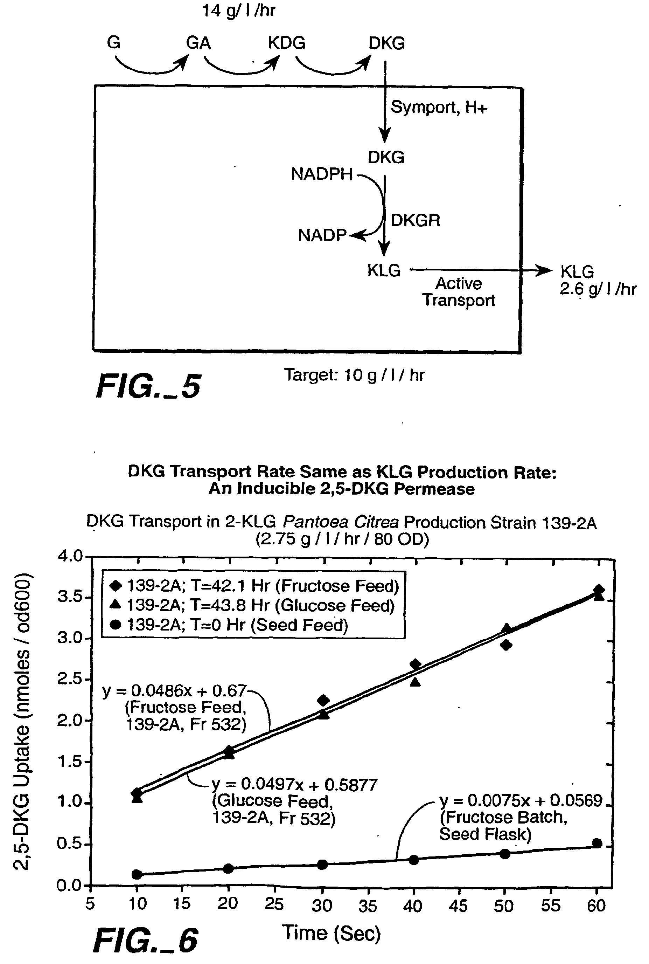

This example narrates the key steps of 2KLG formation from glucose which can be compartmentalized into four parts (Fig. 5). Production of the key intermediate 2,5-DKG using three periplasmic enzymes in P. citrea at 14-15 g/l/hr rate (Sonoyama, et al, Appl.Environ. Microbiol., 1982, 43:1064-1069). The second part is the rate by which DKG is needed to be transported in the cell's cytoplasmic space. The third is the rate of conversion of DKG to 2KLG using DKG reductases (US Patent No. 5,032,514). DKG to 2KLG conversion is not the rate limiting when DKG reductase is overexpressed. When in our current 2KLG production fermentations, inducible plasmids were used to both increase and decrease reductase specific activities relative to our typical fermentations, which presently use pD92, in which DKG reductase in under a constitutive trp promoter. The inducible plasmid, pD23, is under a taq promoter, and can be induced with IPTG. Three fermentors were run, one with pD92, and two with pD23, one of which was induced with IPTG. The control pD92 and the induced pD23 produced nearly identical levels of 2-KLG, while the uninduced pD23 made significantly less. Assays of reductase specific activities show that relative to the control pD92, the uninduced plasmid made less than half the levels of reductase, while the induced pD23 made more than twice as much. These results indicate that the level of reductase activity in our 2KLG producing P. citrea fermentation is not the bottleneck for the production of 2-KLG.

| Strains | Protein Concentration | Reductase Activity | Specific Activity |
|---|---|---|---|
| pD92 (control) | 3.2 mg/ml | 25.0 u/ml | 7.8 u/mg |
| pD23 (uninduced) | 3.7 mg/ml | 12.0 u/ml | 3.2 u/mg |
| pD23 (induced) | 3.9 mg/ml | 73 u/ml | 19 u/mg |

The fourth part is the transport of 2KLG which is intracellularly made and need to be exported out. The production rate of 2KLG in the fermentation ( 2.2 g/l/hr - 2.7 g/l/hr) is considered to be equal to the export rate of 2KLG from the cell. It is argued, if the rate of export of 2KLG is limiting then cells will accumulate 2KLG in side the cell and cells will not be able to function at their metabolic potential and eventually die. However, 2KLG production cells of P. citrea do not exhibit either of these conditions and intracellular measurements of 2KLG remain 10-20 fold below the maximum concentration of 2KLG produced. It is thus conceived that 2KLG export is also not a rate limiting step in the production of 2KLG.

### Example III

Example III provides the proof that indeed the transport rate of substrate in to the cell for bioconversion can be the rate limiting step.

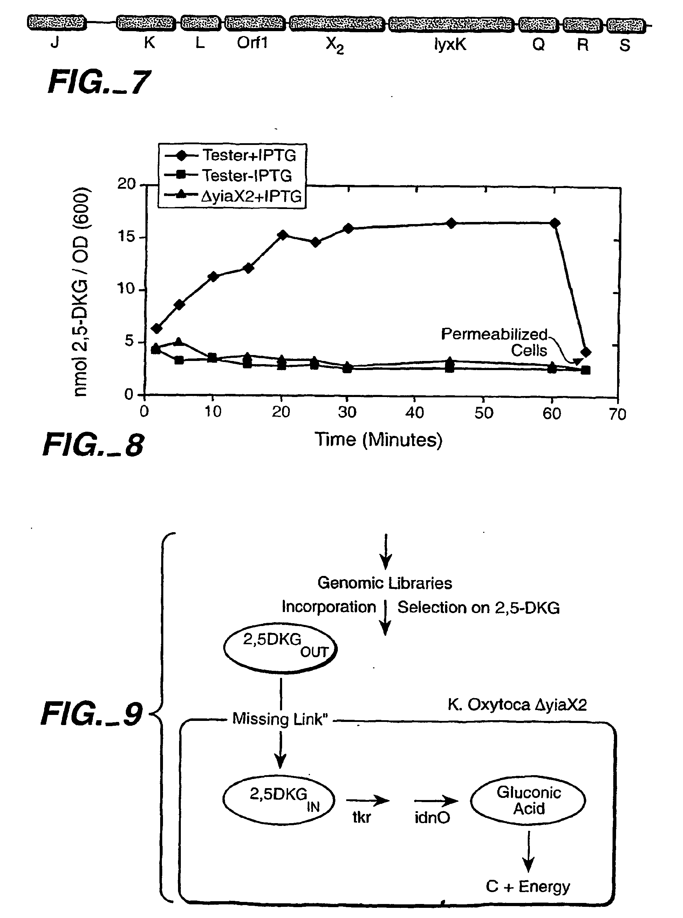

Cell pellets from three different times of the fermentation process, seed-flask stage, fructose feed stage and glucose feed stage were collected and processed as described in the experimental. The DKG uptake assay using these cell-types gave 0.5 g/l/hr, 2.7 g/l/hr and 2.75 g/l/hr DKG uptake rate of bring in the DKG to get converted to KLG (Fig 9). The rate of DKG import is same as KLG export. This result thus demonstrate that the rate of bioconversion of DKG to KLG is dependent upon the import rate of 2,5-DKG into the cell. Thus this invention will demonstrate that by increasing the DKG uptake transporters by overexpression will enhance the import rate of 2,5-DKG and thus will enhance the 2KLG production rate. Those expert in this art, can visualize that the key limiting factor in various biotransformations using whole cell conversion methods may indeed be the import and the import rate of substrate into the cell for the biotransformation.

### Example IV

Example IV provides the discovery of a 2,5-DKG transporter in K. oxytoca using DKG uptake assay. WO 002170 describes the identification and sequencing of an operon from Klebsiella oxytoca, designated the yia operon, which contains eight putative open reading frames. The functions of these polypeptides encoded by the individual open reading frames in the yia operon are not described in WO002170. Disruption of this Operon removed the ability of K. oxytoca to use ascorbic acid as sole carbon source. It is known that ascorbic acid is an oxidatively unstable substance and it decomposes to 2,3-DKG by air oxidation (Kimaya, S., J. Vitaminol., 1961, 7:19-26). It was thus reasonable to suggest that it is 2,3-DKG which is the real substrate for growth. One of the open reading frames in the yia operon , designated as yiaX2, encoded a transporter type transmembrane protein and was thus considered a candidate for 2,3-DKG permease. In light of the parallel search of finding 2,5-DKG permease, it was thus realized that 2,3-DKG and 2,5-DKG being analogous molecules, it may be possible that yiaX2 can transport 2,5-DKG and other sugar keto acids such as 2KLG.

In order to determine whether *yiaX2* can transport 2,5-DKG, and 2-KLG this gene was deleted from the chromosome of K. oxytoca strain M5a1 (see, for example, Streicher et al., Proc. Natl. Acad. Sci. 68: 1174-1177 (1971)). The yiaX2 deletion mutant designated as MGK002 was created using standard molecular biology protocols known in the art (see for example, Hamilton et al., J. bacterial. 171:4617-4622 (1989)). Another K. oxytoca strain designated as Tester strain was created by adding back the plasmidly encoded and lac operon regulated yiaX2 gene to K. oxytoca strain MGK002. DKG uptake assay under +/- IPTG induction using MGK002 and Tester strain confirmed that yiaX2 encoded a poly peptide having 2,5-DKG transport activities (Fig 9).

### Example V

Example V provides the selection methodology for screening 2,5-DKG permeases form microorganisms

With the information that yiaX2 gene encoded a transporter protein having 2,5-DKG transport activities, it became possible to design a selection host for finding 2,5-DKG transporter protein of P. citrea and other biological sources. K. oxytoca with yiaX2 gene deletion and addition of genes to express enzymes involved in the catabolism of 2,5-DKG to gluconic acid, which is assessable to the central metabolism of an organism. Enzymes capable of catabolizing 2,5-DKG to gluconic acid are encoded by tkr and idno genes of the tkr idnD idnO operon present in some Gram negative organisms (Bausch, C., et al, J. Bacteriol., 1998, 180:3704-3710).

The resulting tester strain of K. oxytoca was yiaX2[tkr idno] and had a all the components needed for growth on 2,5-DKG as a sole carbon source except its inability to import DKG into the cytoplasm. Therefore, a nucleic acid molecule that encodes a 2,5-DKG permease, upon expression in the tester strain, should confer the ability of the tester strain to grow on 2,5-DKG. This selection methodology is shown in figure 9.

The cloning vector used for constructing the P. citrea genomic libraries is plasmid pCl1920 (Lemer et al., Nucleic Acid Res., 1994, 18:4621 ), a low-copy number expression vector which carries a spectinomycin/streptomycin resistance determinant. Expression is driven by the lacPO promoter/operator region which is repressed by laclq gene product when provided by the host. Genomic DNA from P. citrea (ATCC 39140) was isolated using standard protocol and genomic library was created (Sambrook, et al, Molecular Cloning: A laboratory manual, Cold Sprint Harbor Laboratory, New York (1992)). The amplified libraries were stored in the form of Plasmid DNA for further use to find 2,5-DKG permease of P. citrea.

### Example VI

Example VI provides the proof that by overexpressing DKG transporter in the host cell, one can enhance the DKG import rate into the cell.

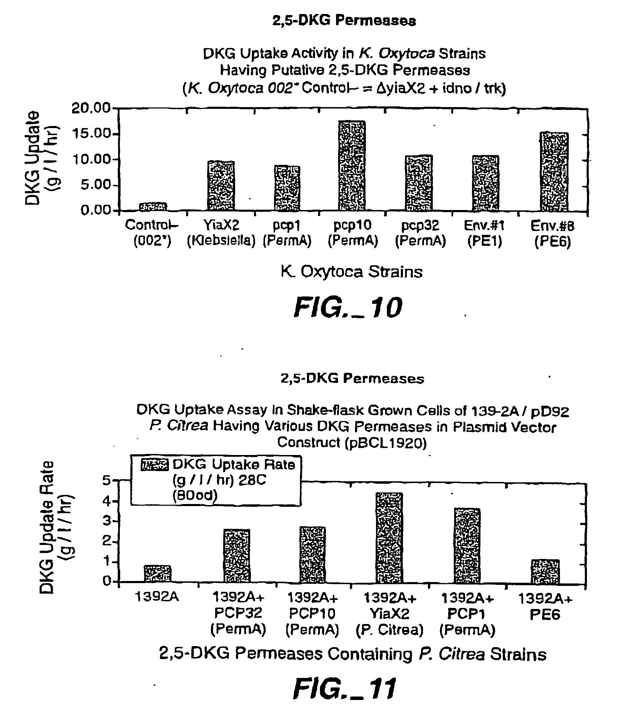

Genomic library was Introduced Into tester strain K. oxytoca yiaX2[tkr [dno] strain. Clones that grew on 2.5-OKG using M9-agar plates with 2.5% 2,5-DKg and 0.1 mM IPTG were tested for DKG uptake using radiolabeled 14C (U) DKG. Various clones were found to have improved DKG uptake than the control tester strain (Fig. 10) Genomic library DNA from these positive clones was transformed into P. citrea (139-2A) and DKG uptake assay was performed to measure the improvement In DKG uptake over the P. citrea 139-2A strain. Three to five fold improvement In DKG uptake rate was seen in the transformants having additional copies of plasmid encoded DKG permeases found through genomic library screening and selection methodology (Fig 11).

### Example VII

Example VII provides the proof that by overexpressing the DKG transporter in the host cell one is able to improve the production of 2KLG.
Nucleic acid molecule encoding Pantoea citrea DKG permease PermA (seq ID no 4) when subcloned using low copy vector pBCL1920 into a Pantoea citrea strain 139-2A suitable for biosynthesis of 2KLG from glucose (US Patent 5,032514), the production rate of 2KLG production improved from 2.5 g/l/hr to 3.2 g/l/hr and yield on the sugar improved from 45 % to 53% (fig 11)

### Example VIII

### Example VIII describes the characteristics of 2,5-DKG permease PermA from P. citrea.

This example describes the membrane topology of PermA of P. citrea. PFAM analysis (Hirokawa, T., et al., Bioinformatics, 1998, 4(4): 378) predicts that the PermA has 11 transmembrane spanning domains, with 8 primary domains and 3 secondary spanning domains (Fig. 13). The amino terminal Is in the periplasm and caboxy terminal being localized in the cytoplasm. Two major and two minor loops exist and both periplasm and cytoplasm have one major and one minor loop. The PermA is a membrane protein with hydrophobicity of 0.62 and has molecular weight of 48 Datton. It is a one H+ assoclated symporter of 2,5-DKG based on the accumulation ratio analysis (Lolkema, J.S., et al. 1996, Handbook of Biological Physics, Chapter 11, 229-260). It belongs to ACS family of MFS based on consensus sequence analysis (Pao, S.S., et al., Microbiol. Molecular Biol. Rev., 1998, 62:1-34)

## Claims

1. A method for enhancing a host cell's biosynthetic production of 2-keto-L-gulonic acid, the method comprising:
a) selecting a host cell that cytosolically converts 2,5-diketo-D-gluconic acid to 2-keto-L-gulonic acid;
b) increasing the transport of said 2,5-diketo-D-gluconic acid into said host cell while maintaining the integrity of the host cell:
c) culturing the host cell to produce said 2-keto-L-gulonic acid; and
d) producing the 2-keto-L-gulonic acid.

2. The method of claim 1, wherein said increasing the transport of 2,5-diketo-D-gluconic acid into said host cell includes the step of transforming into said host cell DNA encoding for one or more proteins transporting said 2,5-diksto-D-gluconic acid into said host cell's cytosolic material.

3. The method of claim 2, wherein said one or more protein is selected from YiaX2 (Fig. 1A-B, Fig 1I), PE1 (Fig. 1C-D), PE6 (Fig. 1E-F), prmA (Fig. 1G) or prmB (Fig. 1H).

4. The method of claim 2, wherein said DNA encoding said protein is capable of hybridization under conditions of intermediate stringency with the nucleic acid sequence encoding YiaX2 (Fig. 1A-B, Fig 1I), PE1 (Fig. 1C-D), PE6 (Fig. 1E-F), prmA (Fig. 1G) or prmB (Fig. 1H).

5. The method of claim 2, wherein said DNA encoding said protein has at least 65% identity with the nucleic acid sequence encoding YiaX2 (Fig. 1A-B, Fig 1I), PE1 (Fig. 1C-D), PE6 (Fig. 1E-F), prmA (Fig. 1G) or prmB (Fig. 1H).

6. The method of claim 5, wherein said DNA encoding said protein has at least 90% identity with the nucleic acid sequence encoding YiaX2 (Fig. 1A-B, Fig 1I), PE1 (Fig. 1C-D), PE6 (Fig. 1E-F), prmA (Fig. 1G) or prmB (Fig. 1H).

7. A method for enhancing the transport of 2,5-diketo-D-gluconic acid into the cytosol across the inner cell membrane, the method comprising:
selecting a host cell; and
transforming into said host cell DNA encoding for one or more proteins capable of transporting 2,5-diketo-D-gluconic acid into said host cell, wherein said one or more protein is selected from YiaX2 (Fig. 1A-B, Fig 1I), PE1 (Fig. 1C-D), PE6 (Fig. 1E-F), prmA (Fig. 1G) or prmB (Fig. 1H).

8. The method of claim 7, wherein said DNA encoding said protein is capable of hybridization under conditions of intermediate stringency with the nucleic acid sequence encoding YiaX2 (Fig. 1A-B, Fig 1I), PE1 (Fig. 1C-D), PE6 (Fig. 1E-F), prmA (Fig. 1G) or prmB (Fig. 1H).

9. The method of claim 7, wherein said DNA encoding said protein has at least 65% identity with the nucleic acid sequence encoding YiaX2 (Fig. 1A-B, Fig 1I), PE1 (Fig. 1C-D), PE6 (Fig. 1E-F), prmA (Fig. 1G) or prmB (Fig. 1H)

10. The method of claim 7, wherein said host cell is selected from the group consisting of bacteria and yeast.

11. The method of claim 10 wherein said host cell is selected from the group consisting of E. coli, Pantoea, and Klebsiella.

## Patentansprüche

1. Verfahren zur Steigerung der biosynthetischen Produktion von 2-Keto-L-gulonsäure durch eine Wirtszelle, wobei das Verfahren folgendes umfasst:
(a) Auswählen einer Wirtszelle, die cytosolisch 2,5-Diketo-D-gluconsäure in 2-Keto-L-gulonsäure umwandelt;
(b) Erhöhen des Transports der 2,5-Diketo-D-gluconsäure in die Wirtszelle unter Beibehalten der Unversehrtheit der Wirtszelle;
(c) Kultivieren der Wirtszelle zur Herstellung der 2-Keto-L-gulonsäure; und (d) Herstellung der 2-Keto-L-gulonsäure.

2. Verfahren von Anspruch 1, wobei das Erhöhen des Transports von 2,5-Diketo-D-gluconsäure in die Wirtszelle den Schritt des Transformierens von DNA in die Wirtszelle umfasst, welche ein oder mehrere Proteine codiert, welche die 2,5-Diketo-D-gluconsäure in das cytosolische Material der Wirtszelle transportieren.

3. Verfahren von Anspruch 2, wobei das eine oder die mehreren Protein(e) gewählt wird aus der Gruppe, bestehend aus YiaX2 (Fig. 1A-B, Fig. 1I), PE1 (Fig. 1CD), PE6 (Fig. 1E-F), prmA (Fig. 1G) oder prmB (Fig. 1H).

4. Verfahren von Anspruch 2, wobei die das Protein codierende DNA zur Hybridisierung unter Bedingungen von mäßiger Stringenz mit der Nukleinsäuresequenz in der Lage ist, welche YiaX2 (Fig. 1A-B, Fig. 1I), PE1 (Fig. 1C-D), PE6 (Fig. 1E-F), prmA (Fig. 1G) oder prmB (Fig. 1H) codiert.

5. Verfahren von Anspruch 2, wobei die das Protein codierende DNA mindestens 65 % Identität mit der Nukleinsäuresequenz aufweist, welche YiaX2 (Fig. 1A-B, Fig. 11), PE1 (Fig. 1C-D), PE6 (Fig. 1E-F), prmA (Fig. 1G) oder prmB (Fig. 1H) codiert.

6. Verfahren von Anspruch 5, wobei die das Protein codierende DNA mindestens 90 % Identität mit der Nukleinsäuresequenz aufweist, welche YiaX2 (Fig. 1A-B, Fig. 1I), PE1 (Fig. 1C-D), PE6 (Fig. 1E-F), prmA (Fig. 1G) oder prmB (Fig. 1H) codiert.

7. Verfahren zur Steigerung des Transports von 2,5-Diketo-D-gluconsäure in das Cytosol über die innere Zellmembran, wobei das Verfahren folgendes umfasst:
Auswählen einer Wirtszelle; und
Transformieren von DNA in die Wirtszelle, welche ein oder mehrere Proteine codiert, welche zum Transport von 2,5-Diketo-D-gluconsäure in die Wirtszelle in der Lage sind, wobei das eine oder die mehreren Protein(e) gewählt wird/werden aus YiaX2 (Fig. 1A-B, Fig. 1I), PE1 (Fig. 1C-D), PE6 (Fig. 1E-F), prmA (Fig. 1G) oder prmB (Fig. 1H).

8. Verfahren von Anspruch 7, wobei die DNA, welche das Protein codiert, zur Hybridisierung unter Bedingungen von mäßiger Stringenz mit der Nukleinsäuresequenz in der Lage ist, welche YiaX2 (Fig. 1A-B, Fig. 1I), PE1 (Fig. 1C-D), PE6 (Fig. 1E-F), prmA (Fig. I G) oder prmB (Fig. 1H) codiert.

9. Verfahren von Anspruch 7, wobei die das Protein codierende DNA mindestens 65 % Identität mit der Nukleinsäuresequenz aufweist, welche YiaX2 (Fig. 1A-B, Fig. 11), PE1 (Fig. 1C-D), PE6 (Fig. 1E-F), prmA (Fig. 1G) oder prmB (Fig. 1H) codiert.

10. Verfahren von Anspruch 7, wobei die Wirtszelle aus der Gruppe gewählt wird, welche aus Bakterien und Hefe besteht.

11. Verfahren von Anspruch 10, wobei die Wirtszelle aus der Gruppe gewählt wird, welche aus E. coli, Pantoea und Klebsiella besteht.

## Revendications

1. Procédé pour améliorer la production biosynthétique d'acide 2-céto-L-gulonique d'une cellule hôte, le procédé comprenant :
a) la sélection d'une cellule hôte qui convertit l'acide 2,5-dicéto-D-gluconique et acide 2-céto-L-gulonique dans son cytosol ;
b) l'augmentation du transport dudit acide 2,5-dicéto-D-gluconique dans ladite cellule hôte tout en maintenant l'intégrité de la cellule hôte ;
c) la mise en culture de la cellule hôte afin de produire ledit acide 2-céto-L-gulonique ; et
d) la production dudit acide 2-céto-L-gulonique.

2. Procédé selon la revendication 1, dans lequel ladite augmentation du transport de l'acide 2,5-dicéto-D-gluconique dans ladite cellule hôte comprend l'étape consistant à transformer ladite cellule hôte avec un ADN codant pour une ou plusieurs protéines transportant ledit acide 2,5-dicéto-D-gluconique dans le matériel cytosolique de ladite cellule hôte.

3. Procédé selon la revendication 2, dans lequel lesdites une ou plusieurs protéines sont sélectionnées parmi YiaX2 (figure 1A-B, figure 1I), PE1 (figure 1C-D), PE6 (figure 1E-F), PermA (figure 1G) et PermB (figure 1H).

4. Procédé selon la revendication 2, dans lequel ledit ADN codant pour ladite protéine est capable de s'hybrider dans des conditions de stringence intermédiaire avec la séquence d'acide nucléique codant pour YiaX2 (figure 1A-B, figure 1I), PE1 (figure 1C-D), PE6 (figure 1E-F), PermA (figure 1G) ou PermB (figure 1H).

5. Procédé selon la revendication 2, dans lequel ledit ADN codant pour ladite protéine présente une identité d'au moins 65 % avec la séquence d'acide nucléique codant pour YiaX2 (figure 1A-B, figure 1I), PE1 (figure 1C-D), PE6 (figure 1E-F), PermA (figure 1G) ou PermB (figure 1H).

6. Procédé selon la revendication 5, dans lequel ledit ADN codant pour ladite protéine présente une identité d'au moins 90 % avec la séquence d'acide nucléique codant pour YiaX2 (figure 1A-B, figure 1I), PE1 (figure 1C-D), PE6 (figure 1E-F), PermA (figure 1G) ou PermB (figure 1H).

7. Procédé pour améliorer le transport d'acide 2,5-dicéto-D-gluconique dans le cytosol à travers la membrane cellulaire interne, le procédé comprenant :
la sélection d'une cellule hôte ; et
la transformation de ladite cellule hôte par un ADN codant pour une ou plusieurs protéines capables de transporter l'acide 2,5-dicéto-D-gluconique dans ladite cellule hôte, lesdites une ou plusieurs protéines étant choisies parmi YiaX2 (figure 1A-B, figure 1I), PE1 (figure 1C-D), PE6 (figure 1E-F), PermA (figure 1G) et PermB (figure 1H).

8. Procédé selon la revendication 7, dans lequel ledit ADN codant pour ladite protéine est capable de s'hybrider dans des conditions de stringence intermédiaire avec la séquence d'acide nucléique codant pour YiaX2 (figure 1A-B, figure 1I), PE1 (figure 1C-D), PE6 (figure 1E-F), PermA (figure 1G) ou PermB (figure 1H).

9. Procédé selon la revendication 7, dans lequel ledit ADN codant pour ladite protéine présente une identité d'au moins 65 % avec la séquence d'acide nucléique codant pour YiaX2 (figure 1A-B, figure 1I), PE1 (figure 1C-D), PE6 (figure 1E-F), PermA (figure 1G) ou PermB (figure 1H).

10. Procédé selon la revendication 7, dans lequel la cellule hôte est choisie dans le groupe consistant en bactéries et levures.

11. Procédé selon la revendication 10 dans lequel la cellule hôte est choisie dans le groupe consistant en *E. coli, Pantoea* et *Klebsiella.*


### SEQUENCE LISTING

<110> Genencor International, Inc.
<120> Enhanced 2-Keto-L-Gluonic Acid Production
<130> GC687-PCTA
<140> PCT/US01/24327
   <141> 2001-08-03
<150> US 09/633,294
   <151> 2000-08-04
<150> US 09/677,032
   <151> 2000-09-29
<160> 12
<170> FastSEQ for Windows Version 4.0
<210> 1
   <211> 1317
   <212> DNA
   <213> Klebsiella oxytoca
<400> 1
   atgaatataa cctctaactc tacaaccaaa gatataccgc gccagcgctg gttaagaatc 60
   attccgccta tactgatcac ttgtattatt tcttatatgg accgggtcaa tattgccttt 120
   gcgatgcccg gaggtatgga tgccgactta ggtatttccg ccaccatggc ggggctggcg 180
   ggcggtattt tctttatcgg ttatctattt ttacaggttc ccggcgggaa aattgccgtt 240
   cacggtagcg gtaagaaatt tatcggctgg tcgctggtcg cctgggcggt catctccgtg 300
   ctgacggggt taattaccaa tcagtaccag ctgctggccc tgcgcttctt actgggcgtg 360
   gcggaaggcg gtatgctgcc ggtcgttctc acgatgatca gtaactggtt ccccgacgct 420
   gaacgcggtc gcgccaacgc gattgtcatt atgtttgtgc cgattgccgg gattatcacc 480
   gccccactct caggctggat tatcacggtt ctcgactggc gctggctgtt tattatcgaa 540
   ggtttgctct cgctggttgt tctggttctg tgggcataca ccatctatga ccgtccgcag 600
   gaagcgcgct ggatttccga agcagagaag cgctatctgg tcgagacgct ggccgcggag 660
   caaaaagcca ttgccggcac cgaggtgaaa aacgcctctc tgagcgccgt tctctccgac 720
   aaaaccatgt ggcagcttat cgccctgaac ttcttctacc agaccggcat ttacggctac 780
   accctgtggc tacccaccat tctgaaagaa ttgacccata gcagcatggg gcaggtcggc 840
   atgcttgcca ttctgccgta cgtcggcgcc attgctggga tgttcctgtt ttcctccctt 900
   tcagaccgaa ccggtaaacg caagctgttc gtctgcctgc cgctgattgg cttcgctctg 960
   tgcatgttcc tgtcggtggc gctgaaaaac caaatttggc tctcctatgc cgcgctggtc 1020
   ggctgcggat tcttcctgca atcggcggct ggcgtgttct ggaccatccc ggcacgtctg 1080
   ttcagcgcgg aaatggcggg cggcgcgcgc ggggttatca acgcgcttgg caacctcggc 1140
   ggattttgtg gcccttatgc ggtcggggtg ctgatcacgt tgtacagcaa agacgctggc 1200
   gtctattgcc tggcgatctc cctggcgctg gccgcgctga tggcgctgct gctgccggcg 1260
   aaatgcgatg ccggtgctgc gccggtaaag acgataaatc cacataaacg cactgcg 1317
<210> 2
   <211> 1281
   <212> DNA
   <213> Unknown
<220>
   <223> Environmental permease PE1
<400> 2
   atgaacagct ctaccaatgc aacgaaacgc tggtggtaca tcatgcctat cgtgtttatc 60
   acgtatagcc tggcgtatct cgaccgcgca aacttcagct ttgcttcggc agcgggcatt 120
   acggaagatt taggcattac caaaggcatc tcgtcgcttc ttggcgcact tttcttcctc 180
   ggctatttct tcttccagat cccgggggcg atttacgcgg aacgccgtag cgtacggaag 240
   ctgattttca tctgtctgat cctgtggggc gcctgcgcct cgcttgaccg ggatggtgca    300
   caatattcca gcgctggctg gcgatccgtt ttattctcgg ctgtcgtgga agcggcggtc    360
   atgccggcga tgctgattta catcagtaac tggtttacca aatcagaacg ttcacgcgcc    420
   aacaccttct taatcctcgg caacccggtc acggtactgt ggatgtcggt ggtctccggc    480
   tacctgattc agtccttcgg ctggcgtgaa atgtttatta ttgaaggcgt tccggccgtc    540
   ctctgggcct tctgctggtg ggtgctggtc aaagttaaac cgtcgcaggt gaactggttg    600
   tcggaaaacg agaaagccgc gctgcaggcg cagctggaga gcgagcagca gggtattaaa    660
   gccgtgcgta actacggcga agccttccgc tcacgcaacg tcattctact gtgcatgcag    720
   tattttgcct ggagtatcgg cgtgtacggt tttgtgctgt ggttgccgtc aattattcgc    780
   agcggcggcg tcaatatggg gatggtggaa gtcggctggc tctcttcggt gccttatctg    840
   gccgcgacta ttgcgatgat cgtcgtctcc tggcttccgg ataaaatgca gaaccgtaaa    900
   ctgttcgtct ggccgctgct gctgattggc ggactggctt ttattggctc atgggccgtc    960
   ggcgctaacc atttctgggc ctcttatacc ctgctggtga ttgccaatgc ggcaatgtac   1020
   gccccttacg gtccgttttt cgccatcatt ccggaaatgc tgccgcgtaa cgtcgccggt   1080
   ggcgcaatgg cgctcatcaa cagcatgggg gccttaggtt cattctttgg ttcgtggttc   1140
   gtgggctacc tgaacggcac caccggcagt ccatcagcct catacatttt catgggagtg   1200
   gcgcttttcg cctcggtatg gcttacttta attgttaagc ctgctaacaa tcaaaagctc   1260
   cccatcggcg ctcgtcacgc c                                              1281
<210> 3
   <211> 1278
   <212> DNA
   <213> Unknown
<220>
   <223> Environmental permease PE6
<400> 3
   atgaatacag cctctgtttc tgtcacccaa agccaggcga tccccaaatt acgctggttg     60
   agaatagtgc cgcctattct tattacctgc attatttcct atatggaccg ggtgaacatc    120
   gccttcgcca tgcccggcgg catggacgat gaactgggca tcaccgcctc gatggccggg    180
   ttggccggcg gtattttctt tatcggttat ctgttcttgc aggtacccgg cggcaagctg    240
   gcggtgtacg gcaacggcaa gaaattcatc ggttggtcgt tgttggcctg ggcggtgatt    300
   tccgtgctga ccgggctggt cacgaatcag tatcaattgc tgttcctgcg cttcgccctc    360
   ggccgtttcc gaagcggcat gctgcggtgg gtgctgacca tgatcagcaa ctggttcccg    420
   gacaaggaac gcgggcgcgc caacgccatc gtcatcatgt tcgtgccgat cgccggcatc    480
   cttaccgcac cgctgtccgg ctggatcatc accgcctggg actggcgcat gctgttcctg    540
   gtcgagggcg cgctgtcgct ggtcgtgatg gtgctgtggt atttcaccat cagcaaccgt    600
   ccacaagagg ccaaaaggat ttcgcaggcg gaaaaagatt atctgatcaa aacgctgcac    660
   gacgaacagt tgctgatcaa aggcaaaacg gtgcgcaacg cctcgctgcg tcgggtgctg    720
   ggcgacaaaa tcatgtggaa gttcttctac cagaccggga tatacggcta caccctgtgg    780
   ctgccgacca ttctcaaggg gctcaccaac ggcaatatgg agcaggtcgg gatgctggct    840
   atcctgccct atatcggcgc catcttcggc atgctgatca tttccaccct ctccgatcgc    900
   accggcaagc gcaaagtgtt cgtcgcactg ccgctggcct gctttgccat ctgcatggcg    960
   ctgtcggtgc tgctgaagga tcacatctgg tggtcgtacg cggcgctggt gggctgtggc   1020
   gtctttaccc aggccgccgc cggggtgttc tggaccattc cgcccaagct gtttaacgcc   1080
   gaaatggccg gcggcgcgcg cggcgtgatc aatgcactgg gcaacctcgg cggtttctgc   1140
   ggcccctata tggtcggcgt gttgatcacc ttgttcagca aagacgtcgg cgtttacagc   1200
   ctcgcggtgt cgctggcctc cgcctcggtg ctggcgttga tgctgccgaa cagatgcgac   1260
   caaaaagcgg gggccgaa                                                 1278
<210> 4
   <211> 1308
   <212> DNA
   <213> Pantoea citrea
<400> 4
   atgcaaaaat cacagccggg aacccgctgg tttcggatta ttgtgccgat cctgatagcc 60
   tgcatcatgt cgtttatgga tcgggtaaat atcagtttcg cattgccggg cggtatggag 120
   caggatctgc tgatgtccag ccagatggcc ggggtagtta gcggtatttt ctttattggt 180
   tatctgtttt tgcaggttcc tggtgggcat atcgcagtac gtggcagtgg taaacgtttt 240
   attgcctggt cgcttgttgc ctgggccgtt gtttctgtcg ctaccgggtt tgtgactcat 300
   cagtaccagc tgttgatttt acgttttgca ctgggggtct ctgaaggtgg gatgttgccg 360
   gtagttctga caatggtcag caactggttt cctgaaaaag agctggggcg tgctaatgca 420
   tttgtcatga tgttcgcccc gcttggcgga atgattaccg cccctgtctc cggatggatt 480
   attgcactgc tagactggcg ctggttattt attatcgaag gattactgtc ggtagtggtt 540
   ctggcagtct ggtggctgat ggtcagtgac cgccctgaag atgcccgttg gctgccggca 600
   gcagaacggg aatatctgct gcgcgaaatg gcccgtgaca aggccgagcg gagcaaactc 660
   cctccgatca gtcatgctcc cctgcaagag gttttccata acccgggcct gatgaagtta 720
   gtgattctga actttttcta tcagacaggt gattacggat acactctgtg gctgccgact 780
   attatcaaaa acctgaccgg agctagtatt ggtaacgtcg gtttgctgac agtgctacct 840
   tttatcgcga cgttatcagg gatttatgtc gtctcttacc tgagcgataa aaccggcaaa 900
   cgtcggcaat gggtgatgat ttctctgttc tgttttgcgg cctgcctgtt ggcctcagtc 960
   ctgttacgtg aatttgtgct ggctgcttat ctggctctgg tggcttgcgg ctttttcctg 1020
   aaagcagcca ccagcccgtt ctggagtatt ccgggacgta ttgcaccgcc ggaagcagcc 1080
   ggtagtgccc gtggtgtaat taacggactg gggaatctgg gcggtttctg cggcccctgg 1140
   ctggtcggat taatgatcta cctgtacgga cagaatgcag ccgttgttac tctggcaggc 1200
   tctctgatca ttgccgggat tattgcggca ttactgccaa cgcagtgtga tctgcgcccg 1260
   gcagaggcac ggcagcagaa tttcacccca cgtattcatg atgccaaa 1308
<210> 5
   <211> 1242
   <212> DNA
   <213> Pantoea citrea
<400> 5
   atgccggtga tttttattac ttacagcctg gcatatctgg atcgggccaa ctacggcttt 60
   gctgctgcct ctgggattga agcagatctt ggaattagcc gtggcacctc ctctctgatt 120
   ggagcactgt tctttctcgg ctacttcatt tttcaggtgc ccggggcaat ttatgcagtg 180
   aaacgcagtg tccgtaaact ggtgtttacc agcctgctgt tgtggggatt ttgtgccgct 240
   gcgaccggac ttatcagcaa tattccggct ctgatggtga tccgctttgt tctgggtgtt 300
   gttgaagccg cagtgatgcc agcgatgctg atttacatca gcaactggtt cacccgtcag 360
   gaacgttcac gggctaatac ctttctggta ttaggtaacc cggtcacggt gttatggatg 420
   tctattgttt ccggatatct gatcaatgct tttggctggc gggaaatgtt tattttcgag 480
   ggtgtgcctg ccttaatctg ggccatcttc tggtggttta ttgtccggga caaaccggag 540
   caggtgagct ggctgacaga aacagaaaag cagcaactgg ccagtgcaat ggctgaagag 600
   cagcaggcaa taccaccgat gcgcaatgtg ccgcaggccc tgcgttcccg caatgtggtg 660
   gtactgtgcc tgttacacgc tctgtggagc atcggagtgt atggttttat gatgtggatg 720
   ccatcgatac tgcgtagcgc tgcatcaatg gacattgtcc gggtaggctg gctggccgca 780
   gttccgtatc tggccgcgat tattactatg ctggtgattt catggctgtc agataaaacc 840
   gggctgcgtc ggctttttat ctggccatta ttgctgattg cgtcagttac tttttttggg 900
   tcctggttac ttgggagcta ctcattctgg ttttcctatg gcttgctggt actggctgct 960
   gcttgtatgt atgccccgta tggaccgttt tttgcgttga ttcctgaatt gctgccaaaa 1020
   aatgtggcgg ggatttctat cgggttaatt aactgttgcg gggcgctggg agcttttgcc 1080
   ggagcctggc tggtgggcta tcttaatggt ctgaccggtg gtccgggggc ttcttacact 1140
   tttatggcca ttgcattgct ggtttctgta gggttggtgt ttttcctgaa agtcccttca 1200
   gggaatttgg tcactcgtcg gttgctgaaa ggtgatgcaa ag 1242
<210> 6
   <211> 1338
   <212> DNA
   <213> Pantoea citrea
<400> 6
   atgaatactc atcaggcagc caaaggtatt gctataccta aacaacggtg gctgagaatt      60
   atctcgccaa taattatcac ctgcattatt tcttatatgg accgggtcaa tattgctttt     120
   gccatgcctg gaggaatgga taaagattta tccgtctctg ccagtatggc cggattggcc     180
   ggcggaatat tttttatagg ttatctgttc ttacaggttc ccggtggaaa aatagccgta     240
   cacggtagtg gtaagaaatt tattggctgg tcactggtgg cctgggcagt aatttcagtt     300
   ctgaccggca tggttactaa ccagtatcag ttgctgtttt tacgctttct tctgggggta     360
   tcagaagggg gtatgttgcc tgttgttctc actatcatca gtaactggtt tccagatcgt     420
   gaacgcggca gagccaactc aatcgtgatt atgtttgtac ccatcgccgg aataatctcc     480
   gccccgctgg caggctggtt aatctcttct ctggactggc gctggctgtt ttatattgaa     540
   ggtttacttt ctctggcggt actgctgctg tgggcactga ctatttctga ccgccccgcc     600
   gaagcacgct ggatctcccg ggccgaaaaa gattatctgc tcaaaacctt gcgggaagag     660
   cagatagctc accaaccccc tttgcgtaaa gtcactctgt cgtcagtgct ggaaaacaga     720
   actttatggc tgctgattgc acttaatttt ttctatcagg ccggaatcta tggctacaca     780
   ttgtggctgc cgaccatttt aaaagatatg gcgcatagca gcatgtccct ggtcggttta     840
   ctggcaattc tgccttatgt cgccgccatg gccggtatgt tcctgttttc ccggttatcg     900
   gataagagtg gcaaacggcg ccgttatgtc attctgccct tgttcggatt cgcgttatgc     960
   atggcattgt cagtaatcag ccagggccat ttatggattt cttacagcgc actgattggc    1020
   tgtggttttt tcctgcagtc agctgctggg gtgttttggg caattccggc ccggttgttt    1080
   agtgccgagg tcgcaggcag tgcccgtggg gtaattaatg ccttaggtaa tcttggcggt    1140
   ttttgtggcc cctatctggt cggcatcttt attacttttt atagccaggc ggccggtgtc    1200
   tatttcctgg caatttcgct ggcgattgcc ggagctctgg cattttgcct gccacgccgc    1260
   tgcgatatgt cagccagcga aatcgccgct gaagatgcaa agattgaacc gctgcccgga    1320
   catgccggga gggtatta                                                  1338
<210> 7
   <211> 439
   <212> PRT
   <213> Klebsiella oxytoca
<400> 7
   Met Asn Ile Thr Ser Asn Ser Thr Thr Lys Asp Ile Pro Arg Gln Arg
   1               5                   10                  15
   Trp Leu Arg Ile Ile Pro Pro Ile Leu Ile Thr Cys Ile Ile Ser Tyr
               20                  25                  30
   Met Asp Arg Val Asn Ile Ala Phe Ala Met Pro Gly Gly Met Asp Ala
           35                  40                  45
   Asp Leu Gly Ile Ser Ala Thr Met Ala Gly Leu Ala Gly Gly Ile Phe
       50                  55                  60
   Phe Ile Gly Tyr Leu Phe Leu Gln Val Pro Gly Gly Lys Ile Ala Val
   65                  70                  75                  80
   His Gly Ser Gly Lys Lys Phe Ile Gly Trp Ser Leu Val Ala Trp Ala
                   85                  90                  95
   Val Ile Ser Val Leu Thr Gly Leu Ile Thr Asn Gln Tyr Gln Leu Leu
               100                 105                 110
   Ala Leu Arg Phe Leu Leu Gly Val Ala Glu Gly Gly Met Leu Pro Val
           115                 120                 125
   Val Leu Thr Met Ile Ser Asn Trp Phe Pro Asp Ala Glu Arg Gly Arg
       130                 135                 140
   Ala Asn Ala Ile Val Ile Met Phe Val Pro Ile Ala Gly Ile Ile Thr
   145                 150                 155                 160
   Ala Pro Leu Ser Gly Trp Ile Ile Thr Val Leu Asp Trp Arg Trp Leu
                   165                 170                 175
   Phe Ile Ile Glu Gly Leu Leu Ser Leu Val Val Leu Val Leu Trp Ala
               180                 185                 190
   Tyr Thr Ile Tyr Asp Arg Pro Gln Glu Ala Arg Trp Ile Ser Glu Ala
           195                 200                 205
   Glu Lys Arg Tyr Leu Val Glu Thr Leu Ala Ala Glu Gln Lys Ala Ile
       210                 215                 220
   Ala Gly Thr Glu Val Lys Asn Ala Ser Leu Ser Ala Val Leu Ser Asp
   225                 230                 235                 240
   Lys Thr Met Trp Gln Leu Ile Ala Leu Asn Phe Phe Tyr Gln Thr Gly
                   245                 250                 255
   Ile Tyr Gly Tyr Thr Leu Trp Leu Pro Thr Ile Leu Lys Glu Leu Thr
               260                 265                 270
   His Ser Ser Met Gly Gln Val Gly Met Leu Ala Ile Leu Pro Tyr Val
           275                 280                 285
   Gly Ala Ile Ala Gly Met Phe Leu Phe Ser Ser Leu Ser Asp Arg Thr
       290                 295                 300
   Gly Lys Arg Lys Leu Phe Val Cys Leu Pro Leu Ile Gly Phe Ala Leu
   305                 310                 315                 320
   Cys Met Phe Leu Ser Val Ala Leu Lys Asn Gln Ile Trp Leu Ser Tyr
                   325                 330                 335
   Ala Ala Leu Val Gly Cys Gly Phe Phe Leu Gln Ser Ala Ala Gly Val
               340                 345                 350
   Phe Trp Thr Ile Pro Ala Arg Leu Phe Ser Ala Glu Met Ala Gly Gly
           355                 360                 365
   Ala Arg Gly Val Ile Asn Ala Leu Gly Asn Leu Gly Gly Phe Cys Gly
       370                 375                 380
   Pro Tyr Ala Val Gly Val Leu Ile Thr Leu Tyr Ser Lys Asp Ala Gly
   385                 390                 395                 400
   Val Tyr Cys Leu Ala Ile Ser Leu Ala Leu Ala Ala Leu Met Ala Leu
                   405                 410                 415
   Leu Leu Pro Ala Lys Cys Asp Ala Gly Ala Ala Pro Val Lys Thr Ile
               420                 425                 430
   Asn Pro His Lys Arg Thr Ala
           435
<210> 8
   <211> 427
   <212> PRT
   <213> Unknown
<220>
   <223> Environmental permease PE1
<400> 8
   Met Asn Ser Ser Thr Asn Ala Thr Lys Arg Trp Trp Tyr Ile Met Pro
   1               5                   10                  15
   Ile Val Phe Ile Thr Tyr Ser Leu Ala Tyr Leu Asp Arg Ala Asn Phe
               20                  25                  30
   Ser Phe Ala Ser Ala Ala Gly Ile Thr Glu Asp Leu Gly Ile Thr Lys
           35                  40                  45
   Gly Ile Ser Ser Leu Leu Gly Ala Leu Phe Phe Leu Gly Tyr Phe Phe
       50                  55                  60
   Phe Gln Ile Pro Gly Ala Ile Tyr Ala Glu Arg Arg Ser Val Arg Lys
   65                  70                  75                  80
   Leu Ile Phe Ile Cys Leu Ile Leu Trp Gly Ala Cys Ala Ser Leu Asp
                   85                  90                  95
   Arg Asp Gly Ala Gln Tyr Ser Ser Ala Gly Trp Arg Ser Val Leu Phe
               100                 105                 110
   Ser Ala Val Val Glu Ala Ala Val Met Pro Ala Met Leu Ile Tyr Ile
           115                 120                 125
   Ser Asn Trp Phe Thr Lys Ser Glu Arg Ser Arg Ala Asn Thr Phe Leu
       130                 135                 140
   Ile Leu Gly Asn Pro Val Thr Val Leu Trp Met Ser Val Val Ser Gly
   145                 150                 155                 160
   Tyr Leu Ile Gln Ser Phe Gly Trp Arg Glu Met Phe Ile Ile Glu Gly
                   165                 170                 175
   Val Pro Ala Val Leu Trp Ala Phe Cys Trp Trp Val Leu Val Lys Val
               180                 185                 190
   Lys Pro Ser Gln Val Asn Trp Leu Ser Glu Asn Glu Lys Ala Ala Leu
           195                 200                 205
   Gln Ala Gln Leu Glu Ser Glu Gln Gln Gly Ile Lys Ala Val Arg Asn
       210                 215                 220
   Tyr Gly Glu Ala Phe Arg Ser Arg Asn Val Ile Leu Leu Cys Met Gln
   225                 230                 235                 240
   Tyr Phe Ala Trp Ser Ile Gly Val Tyr Gly Phe Val Leu Trp Leu Pro
                   245                 250                 255
   Ser Ile Ile Arg Ser Gly Gly Val Asn Met Gly Met Val Glu Val Gly
               260                 265                 270
   Trp Leu Ser Ser Val Pro Tyr Leu Ala Ala Thr Ile Ala Met Ile Val
           275                 280                 285
   Val Ser Trp Ala Ser Asp Lys Met Gln Asn Arg Lys Leu Phe Val Trp
       290                 295                 300
   Pro Leu Leu Leu Ile Gly Gly Leu Ala Phe Ile Gly Ser Trp Ala Val
   305                 310                 315                 320
   Gly Ala Asn His Phe Trp Ala Ser Tyr Thr Leu Leu Val Ile Ala Asn
                   325                 330                 335
   Ala Ala Met Tyr Ala Pro Tyr Gly Pro Phe Phe Ala Ile Ile Pro Glu
               340                 345                 350
   Met Leu Pro Arg Asn Val Ala Gly Gly Ala Met Ala Leu Ile Asn Ser
           355                 360                 365
   Met Gly Ala Leu Gly Ser Phe Phe Gly Ser Trp Phe Val Gly Tyr Leu
       370                 375                 380
   Asn Gly Thr Thr Gly Ser Pro Ser Ala Ser Tyr Ile Phe Met Gly Val
   385                 390                 395                 400
   Ala Leu Phe Ala Ser Val Trp Leu Thr Leu Ile Val Lys Pro Ala Asn
                   405                 410                 415
   Asn Gln Lys Leu Pro Ile Gly Ala Arg His Ala
               420                 425
<210> 9
   <211> 426
   <212> PRT
   <213> Unknown
<220>
   <223> Environmental permease PE6
<400> 9
   Met Asn Thr Ala Ser Val Ser Val Thr Gln Ser Gln Ala Ile Pro Lys
    1               5                  10                  15
   Leu Arg Trp Leu Arg Ile Val Pro Pro Ile Leu Ile Thr Cys Ile Ile
               20                  25                  30
   Ser Tyr Met Asp Arg Val Asn Ile Ala Phe Ala Met Pro Gly Gly Met
           35                  40                  45
   Asp Asp Glu Leu Gly Ile Thr Ala Ser Met Ala Gly Leu Ala Gly Gly
       50                  55                  60
   Ile Phe Phe Ile Gly Tyr Leu Phe Leu Gln Val Pro Gly Gly Lys Leu
   65                  70                  75                  80
   Ala Val Tyr Gly Asn Gly Lys Lys Phe Ile Gly Trp Ser Leu Leu Ala
                   85                  90                  95
   Trp Ala Val Ile Ser Val Leu Thr Gly Leu Val Thr Asn Gln Tyr Gln
               100                 105                 110
   Leu Leu Phe Leu Arg Phe Ala Leu Gly Arg Phe Arg Ser Gly Met Leu
           115                 120                 125
   Arg Trp Val Leu Thr Met Ile Ser Asn Trp Phe Pro Asp Lys Glu Arg
       130                 135                 140
   Gly Arg Ala Asn Ala Ile Val Ile Met Phe Val Pro Ile Ala Gly Ile
   145                 150                 155                 160
   Leu Thr Ala Pro Leu Ser Gly Trp Ile Ile Thr Ala Trp Asp Trp Arg
                   165                 170                 175
   Met Leu Phe Leu Val Glu Gly Ala Leu Ser Leu Val Val Met Val Leu
               180                 185                 190
   Trp Tyr Phe Thr Ile Ser Asn Arg Pro Gln Glu Ala Lys Arg Ile Ser
           195                 200                 205
   Gln Ala Glu Lys Asp Tyr Leu Ile Lys Thr Leu His Asp Glu Gln Leu
       210                 215                 220
   Leu Ile Lys Gly Lys Thr Val Arg Asn Ala Ser Leu Arg Arg Val Leu
   225                 230                 235                 240
   Gly Asp Lys Ile Met Trp Lys Phe Phe Tyr Gln Thr Gly Ile Tyr Gly
                   245                 250                 255
   Tyr Thr Leu Trp Leu Pro Thr Ile Leu Lys Gly Leu Thr Asn Gly Asn
               260                 265                 270
   Met Glu Gln Val Gly Met Leu Ala Ile Leu Pro Tyr Ile Gly Ala Ile
           275                 280                 285
   Phe Gly Met Leu Ile Ile Ser Thr Leu Ser Asp Arg Thr Gly Lys Arg
       290                 295                 300
   Lys Val Phe Val Ala Leu Pro Leu Ala Cys Phe Ala Ile Cys Met Ala
   305                 310                 315                 320
   Leu Ser Val Leu Leu Lys Asp His Ile Trp Trp Ser Tyr Ala Ala Leu
                   325                 330                 335
   Val Gly Cys Gly Val Phe Thr Gln Ala Ala Ala Gly Val Phe Trp Thr
               340                 345                 350
   Ile Pro Pro Lys Leu Phe Asn Ala Glu Met Ala Gly Gly Ala Arg Gly
           355                 360                 365
   Val Ile Asn Ala Leu Gly Asn Leu Gly Gly Phe Cys Gly Pro Tyr Met
       370                 375                 380
   Val Gly Val Leu Ile Thr Leu Phe Ser Lys Asp Val Gly Val Tyr Ser
   385                 390                 395                 400
   Leu Ala Val Ser Leu Ala Ser Ala Ser Val Leu Ala Leu Met Leu Pro
   405 410 415
   Asn Arg Cys Asp Gln Lys Ala Gly Ala Glu
   420 425
<210> 10
   <211> 436
   <212> PRT
   <213> Pantoea citrea
<400> 10
   Met Gln Lys Ser Gln Pro Gly Thr Arg Trp Phe Arg Ile Ile Val Pro
   1               5                   10                  15
   Ile Leu Ile Ala Cys Ile Met Ser Phe Met Asp Arg Val Asn Ile Ser
               20                  25                  30
   Phe Ala Leu Pro Gly Gly Met Glu Gln Asp Leu Leu Met Ser Ser Gln
           35                  40                  45
   Met Ala Gly Val Val Ser Gly Ile Phe Phe Ile Gly Tyr Leu Phe Leu
       50                  55                  60
   Gln Val Pro Gly Gly His Ile Ala Val Arg Gly Ser Gly Lys Arg Phe
   65                  70                  75                  80
   Ile Ala Trp Ser Leu Val Ala Trp Ala Val Val Ser Val Ala Thr Gly
                   85                  90                  95
   Phe Val Thr His Gln Tyr Gln Leu Leu Ile Leu Arg Phe Ala Leu Gly
               100                 105                 110
   Val Ser Glu Gly Gly Met Leu Pro Val Val Leu Thr Met Val Ser Asn
           115                 120                 125
   Trp Phe Pro Glu Lys Glu Leu Gly Arg Ala Asn Ala Phe Val Met Met
       130                 135                 140
   Phe Ala Pro Leu Gly Gly Met Ile Thr Ala Pro Val Ser Gly Trp Ile
   145                 150                 155                 160
   Ile Ala Leu Leu Asp Trp Arg Trp Leu Phe Ile Ile Glu Gly Leu Leu
                   165                 170                 175
   Ser Val Val Val Leu Ala Val Trp Trp Leu Met Val Ser Asp Arg Pro
               180                 185                 190
   Glu Asp Ala Arg Trp Leu Pro Ala Ala Glu Arg Glu Tyr Leu Leu Arg
           195                 200                 205
   Glu Met Ala Arg Asp Lys Ala Glu Arg Ser Lys Leu Pro Pro Ile Ser
       210                 215                 220
   His Ala Pro Leu Gln Glu Val Phe His Asn Pro Gly Leu Met Lys Leu
   225                 230                 235                 240
   Val Ile Leu Asn Phe Phe Tyr Gln Thr Gly Asp Tyr Gly Tyr Thr Leu
                   245                 250                 255
   Trp Leu Pro Thr Ile Ile Lys Asn Leu Thr Gly Ala Ser Ile Gly Asn
               260                 265                 270
   Val Gly Leu Leu Thr Val Leu Pro Phe Ile Ala Thr Leu Ser Gly Ile
           275                 280                 285
   Tyr Val Val Ser Tyr Leu Ser Asp Lys Thr Gly Lys Arg Arg Gln Trp
       290                 295                 300
   Val Met Ile Ser Leu Phe Cys Phe Ala Ala Cys Leu Leu Ala Ser Val
   305                 310                 315                 320
   Leu Leu Arg Glu Phe Val Leu Ala Ala Tyr Leu Ala Leu Val Ala Cys
                   325                 330                 335
   Gly Phe Phe Leu Lys Ala Ala Thr Ser Pro Phe Trp Ser Ile Pro Gly
               340                 345                 350
   Arg Ile Ala Pro Pro Glu Ala Ala Gly Ser Ala Arg Gly Val Ile Asn
           355                 360                 365
   Gly Leu Gly Asn Leu Gly Gly Phe Cys Gly Pro Trp Leu Val Gly Leu
       370                 375                 380
   Met Ile Tyr Leu Tyr Gly Gln Asn Ala Ala Val Val Thr Leu Ala Gly
   385                 390                 395                 400
   Ser Leu Ile Ile Ala Gly Ile Ile Ala Ala Leu Leu Pro Thr Gln Cys
                   405                 410                 415
   Asp Leu Arg Pro Ala Glu Ala Arg Gln Gln Asn Phe Thr Pro Arg Ile
               420                 425                 430
   His Asp Ala Lys
                           435
<210> 11
   <211> 414
   <212> PRT
   <213> Pantoea citrea
<400> 11
   Met Pro Val Ile Phe Ile Thr Tyr Ser Leu Ala Tyr Leu Asp Arg Ala
   1 5 10 15
   Asn Tyr Gly Phe Ala Ala Ala Ser Gly Ile Glu Ala Asp Leu Gly Ile
   20 25 30
   Ser Arg Gly Thr Ser Ser Leu Ile Gly Ala Leu Phe Phe Leu Gly Tyr
   35 40 45
   Phe Ile Phe Gln Val Pro Gly Ala Ile Tyr Ala Val Lys Arg Ser Val
   50 55 60
   Arg Lys Leu Val Phe Thr Ser Leu Leu Leu Trp Gly Phe Cys Ala Ala
   65 70 75 80
   Ala Thr Gly Leu Ile Ser Asn Ile Pro Ala Leu Met Val Ile Arg Phe
   85 90 95
   Val Leu Gly Val Val Glu Ala Ala Val Met Pro Ala Met Leu Ile Tyr
   100 105 110
   Ile Ser Asn Trp Phe Thr Arg Gln Glu Arg Ser Arg Ala Asn Thr Phe
   115 120 125
   Leu Val Leu Gly Asn Pro Val Thr Val Leu Trp Met Ser Ile Val Ser
   130 135 140
   Gly Tyr Leu Ile Asn Ala Phe Gly Trp Arg Glu Met Phe Ile Phe Glu
   145 150 155 160
   Gly Val Pro Ala Leu Ile Trp Ala Ile Phe Trp Trp Phe Ile Val Arg
   165 170 175
   Asp Lys Pro Glu Gln Val Ser Trp Leu Thr Glu Thr Glu Lys Gln Gln
   180 185 190
   Leu Ala Ser Ala Met Ala Glu Glu Gln Gln Ala Ile Pro Pro Met Arg
   195 200 205
   Asn Val Pro Gln Ala Leu Arg Ser Arg Asn Val Val Val Leu Cys Leu
   210 215 220
   Leu His Ala Leu Trp Ser Ile Gly Val Tyr Gly Phe Met Met Trp Met
   225 230 235 240
   Pro Ser Ile Leu Arg Ser Ala Ala Ser Met Asp Ile Val Arg Val Gly
   245 250 255
   Trp Leu Ala Ala Val Pro Tyr Leu Ala Ala Ile Ile Thr Met Leu Val
   260 265 270
   Ile Ser Trp Leu Ser Asp Lys Thr Gly Leu Arg Arg Leu Phe Ile Trp
   275 280 285
   Pro Leu Leu Leu Ile Ala Ser Val Thr Phe Phe Gly Ser Trp Leu Leu
   290 295 300
   Gly Ser Tyr Ser Phe Trp Phe Ser Tyr Gly Leu Leu Val Leu Ala Ala
   305 310 315 320
   Ala Cys Met Tyr Ala Pro Tyr Gly Pro Phe Phe Ala Leu Ile Pro Glu
   325 330 335
   Leu Leu Pro Lys Asn Val Ala Gly Ile Ser Ile Gly Leu Ile Asn Cys
   340 345 350
   Cys Gly Ala Leu Gly Ala Phe Ala Gly Ala Trp Leu Val Gly Tyr Leu
   355 360 365
   Asn Gly Leu Thr Gly Gly Pro Gly Ala Ser Tyr Thr Phe Met Ala Ile
   370 375 380
   Ala Leu Leu Val Ser Val Gly Leu Val Phe Phe Leu Lys Val Pro Ser
   385 390 395 400
   Gly Asn Leu Val Thr Arg Arg Leu Leu Lys Gly Asp Ala Lys
   405 410
<210> 12
   <211> 446
   <212> PRT
   <213> Pantoea citrea
<400> 12
   Met Asn Thr His Gln Ala Ala Lys Gly Ile Ala Ile Pro Lys Gln Arg
    1              5                   10                  15
   Trp Leu Arg Ile Ile Ser Pro Ile Ile Ile Thr Cys Ile Ile Ser Tyr
               20                  25                  30
   Met Asp Arg Val Asn Ile Ala Phe Ala Met Pro Gly Gly Met Asp Lys
           35                  40                  45
   Asp Leu Ser Val Ser Ala Ser Met Ala Gly Leu Ala Gly Gly Ile Phe
       50                  55                  60
   Phe Ile Gly Tyr Leu Phe Leu Gln Val Pro Gly Gly Lys Ile Ala Val
   65                  70                  75                  80
   His Gly Ser Gly Lys Lys Phe Ile Gly Trp Ser Leu Val Ala Trp Ala
                   85                  90                  95
   Val Ile Ser Val Leu Thr Gly Met Val Thr Asn Gln Tyr Gln Leu Leu
               100                 105                 110
   Phe Leu Arg Phe Leu Leu Gly Val Ser Glu Gly Gly Met Leu Pro Val
           115                 120                 125
   Val Leu Thr Ile Ile Ser Asn Trp Phe Pro Asp Arg Glu Arg Gly Arg
       130                 135                 140
   Ala Asn Ser Ile Val Ile Met Phe Val Pro Ile Ala Gly Ile Ile Ser
   145                 150                 155                 160
   Ala Pro Leu Ala Gly Trp Leu Ile Ser Ser Leu Asp Trp Arg Trp Leu
                   165                 170                 175
   Phe Tyr Ile Glu Gly Leu Leu Ser Leu Ala Val Leu Leu Leu Trp Ala
               180                 185                 190
   Leu Thr Ile Ser Asp Arg Pro Ala Glu Ala Arg Trp Ile Ser Arg Ala
           195                 200                 205
   Glu Lys Asp Tyr Leu Leu Lys Thr Leu Arg Glu Glu Gln Ile Ala His
       210                 215                 220
   Gln Pro Pro Leu Arg Lys Val Thr Leu Ser Ser Val Leu Glu Asn Arg
   225                 230                 235                 240
   Thr Leu Trp Leu Leu Ile Ala Leu Asn Phe Phe Tyr Gln Ala Gly Ile
                   245                 250                 255
   Tyr Gly Tyr Thr Leu Trp Leu Pro Thr Ile Leu Lys Asp Met Ala His
               260                 265                 270
   Ser Ser Met Ser Leu Val Gly Leu Leu Ala Ile Leu Pro Tyr Val Ala
           275                 280                 285
   Ala Met Ala Gly Met Phe Leu Phe Ser Arg Leu Ser Asp Lys Ser Gly
       290                 295                 300
   Lys Arg Arg Arg Tyr Val Ile Leu Pro Leu Phe Gly Phe Ala Leu Cys
   305                 310                 315                 320
   Met Ala Leu Ser Val Ile Ser Gln Gly His Leu Trp Ile Ser Tyr Ser
                   325                 330                 335
   Ala Leu Ile Gly Cys Gly Phe Phe Leu Gln Ser Ala Ala Gly Val Phe
               340                 345                 350
   Trp Ala Ile Pro Ala Arg Leu Phe Ser Ala Glu Val Ala Gly Ser Ala
           355                 360                 365
   Arg Gly Val Ile Asn Ala Leu Gly Asn Leu Gly Gly Phe Cys Gly Pro
       370                 375                 380
   Tyr Leu Val Gly Ile Phe Ile Thr Phe Tyr Ser Gln Ala Ala Gly Val
   385                 390                 395                 400
   Tyr Phe Leu Ala Ile Ser Leu Ala Ile Ala Gly Ala Leu Ala Phe Cys
                   405                 410                 415
   Leu Pro Arg Arg Cys Asp Met Ser Ala Ser Glu Ile Ala Ala Glu Asp
               420                 425                 430
   Ala Lys Ile Glu Pro Leu Pro Gly His Ala Gly Arg Val Leu
           435                 440                 445